# EUROPÄISCHE PATENTANMELDUNG

(11) **EP 0 919 205 A2**
(43) Veröffentlichungstag der Anmeldung: **02.06.1999**
(21) Anmeldenummer: 98121278.0
(22) Anmeldetag: 09.11.1998
(51) Int. Cl.: A61C 1/14

(54) **Medizinisches oder dentalmedizinisches Behandlungsinstrument zur spanabhebenden Behandlung von Körpergewebe oder einem Ersatzstoff**

(30) Priorität: 21.11.1997 DE 19751682
(71) Anmelder: KALTENBACH & VOIGT GMBH & CO., 88400 Biberach (DE)
(72) Erfinder: Löhn, Gerd, 88400 Biberbach-Rissegg (DE); Krause, Gerhard, 88447 Warthausen (DE); Mössle, Walter, 88441 Mittelbiberach (DE)
(74) Vertreter: Schmidt-Evers, Jürgen, Dipl.-Ing.

(57) **Zusammenfassung**

Bei einem medizinischen oder dentalmedizinischen Behandlungsinstrument zur insbesondere spanabhebenden Behandlung von Körpergewebe oder einem Ersatzstoff, mit einem länglichen Handstück (1), das in seinem vorderen Endbereich eine Haltevorrichtung (6) mit einem Steckloch (27) aufweist, in das das Werkzeug (7) mit seinem Werkzeugschaft (8) einsteckbar und mittels eines Sicherungsteiles (29) sicherbar ist, das quer zum Werkzeugschaft (8) zwischen einer Sicherungsstellung, in der es in eine Sicherungsausnehmung (28) im Werkzeugschaft (8) einfaßt, und einer den Werkzeugschaft (8) freigebenden Freigabestellung bewegbar gelagert und durch die Kraft einer Feder in seine Sicherungsstellung beaufschlagt ist, ist ein von außen manuell zugängliches Betätigungsglied (91) vorgesehen, das an der Haltevorrichtung (6) bewegbar gelagert ist und mit dem das Sicherungsteil (29) in seine Freigabestellung verschiebbar ist, wobei das Betätigungsglied (91) quer zum Werkzeugschaft (8) verschiebbar gelagert ist.

## Beschreibung

In der medizinischen oder dentalmedizinischen Technik kann eine Behandlung des menschlichen oder tierischen Körpers oder künstlichen Teilen davon (Prothesen) mit einem Behandlungsinstrument auf verschiedene Weise erfolgen. In vielen Fallen ist lediglich eine Behandlung des Körpers erforderlich, ohne seine Form zu verändern. Hierbei kann es sich z.B. um eine Oberflächenbehandlung handeln, die z.B. mit einem Behandlungsinstrument mit einem besonderen Werkzeug zur Oberflächenbehandlung durchgeführt werden kann, wobei es sich z.B. um eine Massagebehandlung oder einen Auftrag von einem Behandlungsmittel auf den Körper handeln kann. Eine andere Art Behandlung besteht z.B. darin, die Form des Körpers zu verändern, wie es z.B. bei einer spanabhebenden Bearbeitung der Fall ist, bei der z.B. ein Schnitt im Körpergewebe ausgeführt wird. Unter spanabhebende Behandlungen fällt auch die Ausarbeitung einer Kavität mit einem spanabhebenden Werkzeug oder die Oberflächenbehandlung eines Zahns, z.B. um einen Belag zu entfernen. Als spanabhebende Werkzeuge können z.B. Rotationswerkzeuge wie Bohrer und Fräser, Feilen und solche Werkzeuge sein, deren wenigstens eine Arbeitsfläche flächig abrasiv ist, d.h. mit einer Vielzahl von Schneidkanten oder -punkten besetzt ist, die nebeneinander angeordnet sind, wobei das Werkzeug durch einen Vibrationsantrieb schwingend angetrieben wird. Ein solches Behandlungsinstrument oder Werkzeug ist z.B. in der WO 96/14024 beschrieben.

An ein Behandlungsinstrument der vorliegenden Art sind eine Mehrzahl Forderungen gestellt. Eine Forderung besteht darin, daß das Behandlungsinstrument von kleiner bzw. schlanker Bauweise sein soll, damit der Blick auf die Behandlungsstelle nicht oder nur unwesentlich beeinträchtigt wird. Sofern bei einem vorliegenden Behandlungsinstrument mehrere unterschiedliche Behandlungswerkzeuge vorhanden sind, soll die Haltevorrichtung ein handhabungsfreundliches und schnelles Austauschen der Werkzeuge ermöglichen, so daß das Behandlungsinstrument leistungsgerecht für unterschiedliche Behandlungsarten eingesetzt werden kann.

Bei einem aus der WO 96/14024 bekannten Behandlungsinstrument ist zur Halterung des Werkzeugs an einem vorragenden Schaft des Behandlungsinstruments ein Querloch im Schaft vorgesehen, in das der Werkzeugschaft einsteckbar und durch ein Fixierelement gegen ein Herausrutschen sicherbar ist, das zwischen einer in eine Sicherung des Werkzeugschaftes einfassenden Sicherungsstellung und einer den Werkzeugschaft freigebenden Freigabestellung verschiebbar und die Kraft einer Feder in seine Sicherungsstellung vorgespannt ist. In der genannten Druckschrift sind mehrere Ausführungsbeispiel einer Haltevorrichtung für ein Werkzeug beschrieben. U.a. ist es möglich, das Werkzeug um seine Längsachse frei drehbar oder in unterschiedlichen Drehstellungen feststellbar in der Haltevorrichtung zu sichern.

Bei einem aus der vorbekannten DE 44 08 574 A1 entnehmbaren Behandlungsinstrument der vorliegenden Art sind zur Sicherung des Werkzeugs in der Haltevorrichtung des Behandlungsinstruments eine Mehrzahl Kugeln auf dem Umfang der Haltevorrichtung verteilt angeordnet, die mittels einer Innenkonusfläche eines axial verschiebbaren Betätigungselements radial einwärts in eine Sicherungsstellung drückbar sind, in der sie in Kalotten des Werkzeugschaftes einfassen, und nach einer vorherigen axialen Verschiebung des Betätigungsteils beim Herausziehen des Werkzeugschaftes aus der Haltevorrichtung radial nach außen in eine Freigabestellung verdrängbar sind. Es ist auch bereits vorgeschlagen worden, eine Haltevorrichtung so auszugestalten, daß die Sicherungsteile beim axialen Verschieben des Werkzeugs, z.B. um es in die Haltevorrichtung einzufuhren oder daraus herauszuziehen, selbsttätig in ihre den Werkzeugschaft freigebende Freigabestellung verdrängbar sind.

Der Erfindung liegt die Aufgabe zugrunde, ein Behandlungsinstrument der vorliegenden Art so auszugestalten, daß bei Gewährleistung einer Kleinen oder kompakten Bauweise eine handhabungsfreundliche Betätigung beim Sichern bzw. Fixieren und/oder Lösen und/oder Austauschen eines Werkzeugs möglich ist.

Diese Aufgabe wird durch die Merkmale des Anspruchs 1 oder 3 gelöst.

Bei beiden erfindungsgemäßen Ausgestaltungen ist das Betätigungsglied zum Lösen der Haltevorrichtung ein Anbauteil der Haltevorrichtung, das zwischen einer das Werkzeug sichernden Sicherungsstellung und einer es freigebenden Freigabestellung beweglich in die Haltevorrichtung integriert ist. Hierdurch kann das Betätigungsglied aufgrund seiner Integration in die Haltevorrichtung nicht verloren gehen, und es steht in handhabungsfreundlicher Position permanent für eine Betätigung zur Verfügung. Dabei läßt sich im Rahmen der Erfindung eine Kleine Bauweise verwirklichen, so daß der Blick auf die Behandlungsstelle nicht oder nur unwesentlich beeinträchtigt ist, und die außerdem handhabungsfreundlich bedienbar ist, wodurch die Brauchbarkeit des Behandlungsinstruments und dessen Eignung auch für unzugängliche Behandlungsstellen verbessert wird, wie es insbesondere im Mundraum eines Patienten der Fall ist.

Außerdem ermöglichen beide erfindungsgemäßen Ausgestaltungen ein handhabungsfreundliches Sichern und/oder Lösen des Werkzeugs und/oder Austauschen unterschiedlicher Werkzeuge, wobei das Betätigungsgsglied bequem betätigt oder ergriffen werden kann. Es kann auch das Werkzeug leicht aus dem Steckloch herausgeschoben bzw. herausgezogen werden, wobei bei der erfindungsgemäßen Ausgestaltung nach Anspruch 2 im Anfangsbereich dieser Bewegung das Sicherungsteil mit dem Betätigungsglied aus der Sicherungsausnehmung bewegt oder verdrängt werden kann. Letzteres ist insbesondere dann leichtgängig, wenn ein Untersetzungsgetriebe oder ein Keilflächengetriebe dazwischengeschaltet ist.

Die Erfindung bezieht sich gemäß Anspruch 30 auch auf ein Werkzeug vorzugsweise mit wenigstens einer abrasiven Arbeitsfläche, die durch einen Schwingungsantrieb aktivierbar ist, wobei der Schaft des Werkzeugs Halterungsausnehmungen aufweist, die bei Gewährleistung einer einfachen und kostengünstigen Herstellung sowie einer handhabungsfreundlichen Bestückung oder Entnahme des Werkzeugs eine vorteilhafte axiale Halterung und gegebenenfalls auch eine Drehstellungs-Positionierung des Werkzeugs gewährleisten.

In den Unteransprüchen sind Merkmale enthalten, die das Behandlungsinstrument bzw. die Haltevorrichtung im Sinne einer kleinen, kostengünstig herstellbaren und handhabungsfreundlich bedienbaren Bauweise weiter verbessern und dabei eine sichere Aufnahme und Halterung des Werkzeugs in der Haltevorrichtung sowie dessen Ausstoß aus der Haltevorrichtung zum Lösen oder Austauschen eines Werkzeugs ermöglichen.

Nachfolgend werden die Erfindung und weiter durch sie erzielbare Vorteile anhand von bevorzugten Ausgestaltungen und Zeichnungen näher erläutert. Es zeigt
- Fig. 1: ein erfindungsgemäßes Behandlungsinstrument zur Behandlung von Körpergewebe oder einem Ersatzstoff im axialen Schnitt;
- Fig. 2: die in Fig. 1 mit X gekennzeichnete Einzelheit in vergrößerter Darstellung;
- Fig. 3: die Einzelheit gemäß Fig. 2 in vereinfachter schematischer Darstellung;
- Fig. 4: die in Fig. 1 mit Y gekennzeichnete Einzelheit, nämlich eine im vorderen Endbereich des Behandlungsinstruments angeordnete Haltevorrichtung für ein Werkzeug im axialen Schnitt;
- Fig. 5: den vorderen Endbereich eines Behandlungsinstruments in abgewandelter Ausgestaltung im axialen Schnitt;
- Fig. 6: einen Handstückschaft des Behandlungsinstruments nach Fig. 5 in der Draufsicht;
- Fig. 7: die in Fig. 1 mit Y gekennzeichnete Einzelheit, nämlich eine Haltevorrichtung für ein Behandlungs-Werkzeug im vorderen Endbereich des Behandlungsinstruments im axialen Schnitt in abgewandelter Ausgestaltung;
- Fig. 8: das vordere Ende eines Handstückschaftes der Haltevorrichtung nach Fig. 7 in teilweise abgwandelter Ausgestaltung im axialen Schnitt;
- Fig. 9: den Handstuckschaft nach Fig. 8 in der Draufsicht;
- Fig.10: die in Fig. 1 mit Y gekennzeichnete Einzelheit, nämlich eine im vorderen Endbereich des Behandlungsinstruments angeordnete Haltevorrichtung für ein Werkzeug im axialen Schnitt in weiter abgewandelter Ausgestaltung;
- Fig.11: eine der Fig. 10 entsprechende Ansicht in weiter abgewandelter Ausgestaltung;
- Fig.12: den Schnitt XII-XII in Fig. 11;
- Fig.13: einen Längsabschnitt eines erfindungsgemäßen Behandlungsinstruments in abgewandelter Ausgestaltung im axialen Schnitt;
- Fig.14: den Längsabschnitt nach Fig. 13 in der Draufsicht.

Die Hauptteile des allgemein mit 1 bezeichneten Behandlungsinstruments sind ein längliches oder stabförmiges Handstück 2 mit einer Griffhülse 3, in der ein vorzugsweise längliches oder stabförmiges Schwingteil 4 schwingbar gelagert und durch einen Schwingungserreger 5 in Schwingungen versetzbar bzw. antreibbar ist, und eine Haltevorrichtung 6 für wenigstens ein Behandlungs-Werkzeug 7, das einen Werkzeugschaft 8 aufweist, der lösbar mit der Haltevorrichtung 6 verbindbar ist.

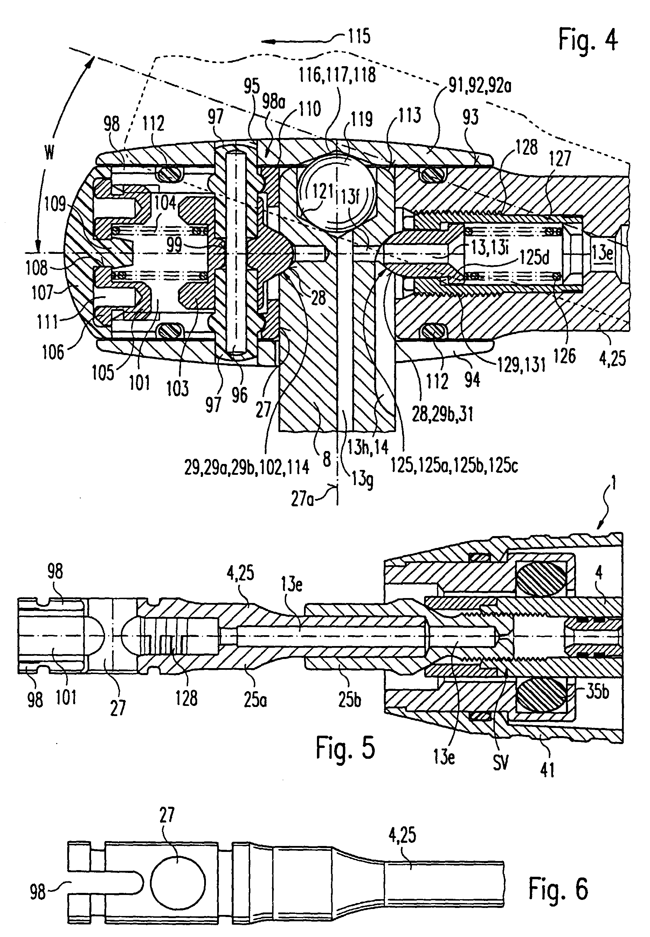

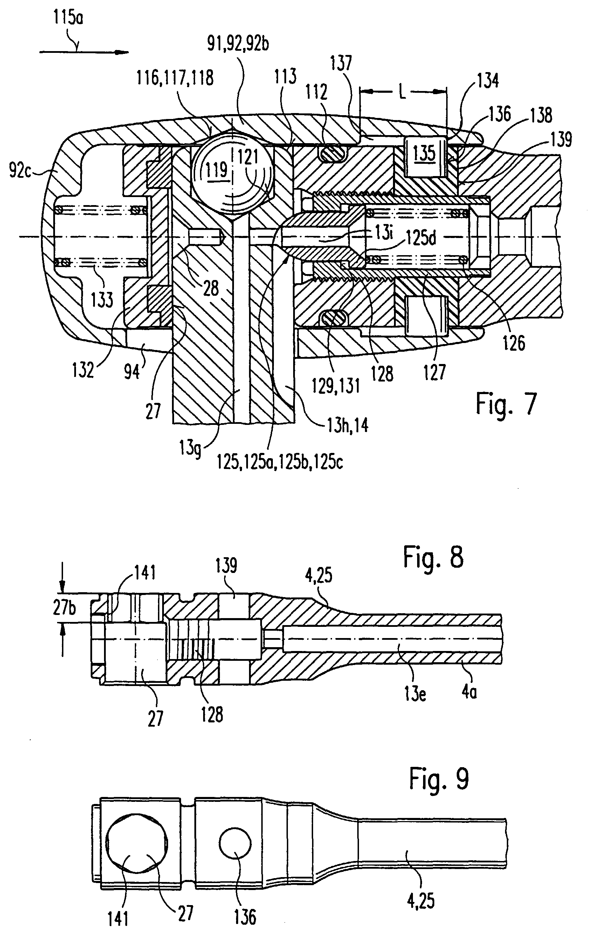

Es ist vorteilhaft, das Behandlungsinstrument 1 mit wenigstens einer, vorzugsweise mehreren sogenannten Medienleitungen auszugestalten, die der Zuführung von Antriebsenergie und Behandlungsmedien wie z.B. einer Behandlungsflüssigkeit und/oder Druckluft und der Zuführung von Licht zur Behandlungsstelle dienen. Bei der vorliegenden Ausgestaltung ist eine Lichtleitung 9 vorgesehen, die sich längs durch das Handstück 2 bis in dessen vorderen Endbereich erstreckt und an einer auf die Behandlungsstelle gerichteten Ausmündungsöffnung 11 ausmündet, und eine Beleuchtungseinrichtung 12 bildet. Außerdem ist eine sich längs durch das Handstück 2 erstreckende Leitung 13 für eine Behandlungs- oder Kühlflüssigkeit, hier Wasser, vorgesehen, die ebenfalls an einer im vorderen Endbereich des Handstücks 2 angeordneten und auf die Behandlungsstelle gerichteten Ausmündungsöffnung 14 (Fig. 4 und 7) ausmündet und eine Zuführungsvorrichtung bildet.

Ferner ist eine Zuführungsleitung 15 für Antriebsenergie vorgesehen, die sich längs durch das Handstück 2 bis zum Antrieb des Schwingungserregers 5 erstreckt. Bei der vorliegenden Ausgestaltung ist ein durch Druckluft antreibbarer Schwingungserreger 5 vorgesehen und die Zuführungsleitung 15 ist eine Druckluftleitung, die sich zum Schwingungserreger 5 erstreckt.

Das Handstück 2 ist durch eine Schnellkupplung oder durch eine Schraub- oder Steckkupplung, insbesondere durch eine frei drehbare Steck-Drehkupplung 16, rückseitig mit einem angedeuteten Anschlußstück 17 lösbar verbindbar, das durch eine flexible Versorgungsleitung 18 mit einer nicht dargestellten Versorgungs- und Steuereinrichtung verbunden ist. Die Steck-Drehkupplung 16 ist bei der vorliegenden Ausgestaltung durch eine hohlzylindrische oder stufenhohlzylindrische Kupplungsausnehmung 16a und einen darin mit Bewegungsspiel einsteckbaren zylindrischen oder stufenzylindrischen Kupplungszapfen 16b gebildet, wobei bei der vorliegenden Ausgestaltung die Kupplungsausnehmung 16a im hinteren Bereich des Handstücks 2 angeordnet ist und der Kupplungszapfen 16b vom Anschlußstück 17 vorragt. Die Medienleitungen durchsetzen die Steck-Drehkupplung 16, so daß bei einem wahlweisen Trennen des Handstücks 2 vom Anschlußstück 17 die Medienleitungen selbsttätig unterbrochen werden. Die Lichtleitung 9 durchsetzt die Steck-Drehkupplung 16 koaxial. Dabei kann ein hinterer Lichtleitungsabschnitt sich koaxial in einer entsprechenden Aufnahmebohrung bis zum vorderen Endbereich des Kupplungszapfens 16b erstrecken an den sich mit einer Trennfuge ein vorderer Lichtleitungsabschnitt anschießt, der sich bis zur Ausmündungsöffnung 14 erstreckt. Der vordere Lichtleitungsabschnitt ist vorzugsweise durch einen Lichtleiter 9a aus Licht leitendem Material, wie z.B. Glas oder Kunststoff, gebildet, wobei es sich um einen flexiblen Lichtleiter oder um einen starren Lichtleiter 9a in Form eines Formstücks handeln kann. Die Querschnittsform des Lichtleiters 9a ist vorzugsweise hinten rund und weiter vorne elipsenförmig. Der hintere Lichtleitungsabschnitt kann ebenfalls durch einen nicht dargestellten Lichtleiter oder durch eine elektrische Leitung mit einer im vorderen Endbereich des Kupplungszapfens 16b angeordnete Lampe 19 gebildet sein, die in einer vorderseitig offenen Ausnehmung vorzugsweise versenkt angeordnet ist.

Die beiden anderen Medienleitungen 13, 15 durchsetzen die hohlzylindrische Trennfuge 21 zwischen dem Kupplungszapfen 16b und der Wandung der Kupplungsausnehmung 16a Z-förmig radial von innen nach außen, wobei die jeweils in der Mantelfläche des Kupplungszapfens 16a und der Innenmantelfläche der Kupplungsausnehmung 16b vorhandenen Öffnungen in einer Ringnut 22 in der Mantelfläche oder in der Innenmantelfläche liegen. Hierdurch ist der Mediendurchgang in jeder Drehstellung der Steck-Drehkupplung 16 auch über 360° hinaus gewährleistet. Die Z-förmigen Durchdringungsstellen sind jeweils durch einen Dichtungsring 23, insbesonder einen O-Ring, abgedichtet, der zu beiden Seiten der Z-förmigen Durchdringungsstelle in einer Ringnut in der Mantelfläche des Kupplungszapfens 16b oder in der Innenmantelfläche der Kupplungsausnehmung 16a angeordnet ist. Rückseitig können am Anschlußstück 17 Anschlüsse, z.B. Anschlußhülsen 24, für in der flexiblen Versorgungsleitung 18 verlaufende Medienleitungsabschnitte angeordnet sein.

Die Haltevorrichtung 6 für das Werkzeug 7 ist im vorderen Endbereich des Schwingteils 4 angeordnet. Bei der vorliegenden Ausgestaltung ist das Schwingteil 4 ein stab- oder hülsenförmiger Körper, der in der vorderen Hälfte des Handstücks 2 angeordnet ist und einen Handstückschaft 25 bildet, der vom vorderen Ende des Handstücks 2 vorragt und sich gerade erstrecken kann oder bezüglich der Längsmittelachse 26 des Handstücks 2 einen spitzen Winkel W von etwa 10 bis 30°, insbesondere etwa 20°, einschließen kann. Die Haltevorrichtung 6 weist ein sich quer oder vorzugsweise rechtwinklig zum Handstückschaft 25 erstreckendes Steckloch 27 auf, das vorzugsweise ein Sackloch ist und somit nur einseitig offen ist, und in das der Werkzeugschaft 8 mit geringem Bewegungsspiel einsteckbar ist. Zur Sicherung des Werkzeugs 7 in der eingesteckten Stellung ist im Werkzeugschaft 8 eine Sicherungsausnehmung 28 vorgesehen, in die ein in der Längsrichtung des Handstückschaftes 25 verstellbares Sicherungsteil 29 hineinbewegbar ist, das zwischen seiner in die Sicherungsausnehmung 28 hineinbewegten Sicherungsstellung und einer herausbewegten, den Werkzeugschaft 8 freigebenden Freigabestellung verstellbar ist und vorzugsweise durch die Kraft einer Feder in seine Sicherungsstellung beaufschlagt ist. Bei der Sicherungsausnehmung 28 kann es sich um eine Ringnut handeln. Es können auch mehrere, auf den Umfang gleichmäßig verteilt angeordnete Kalotten vorgesehen sein, die vorzugsweise kegelförmige Einsenkungen mit einer zentralen Kern-Freibohrung aufweisen können.

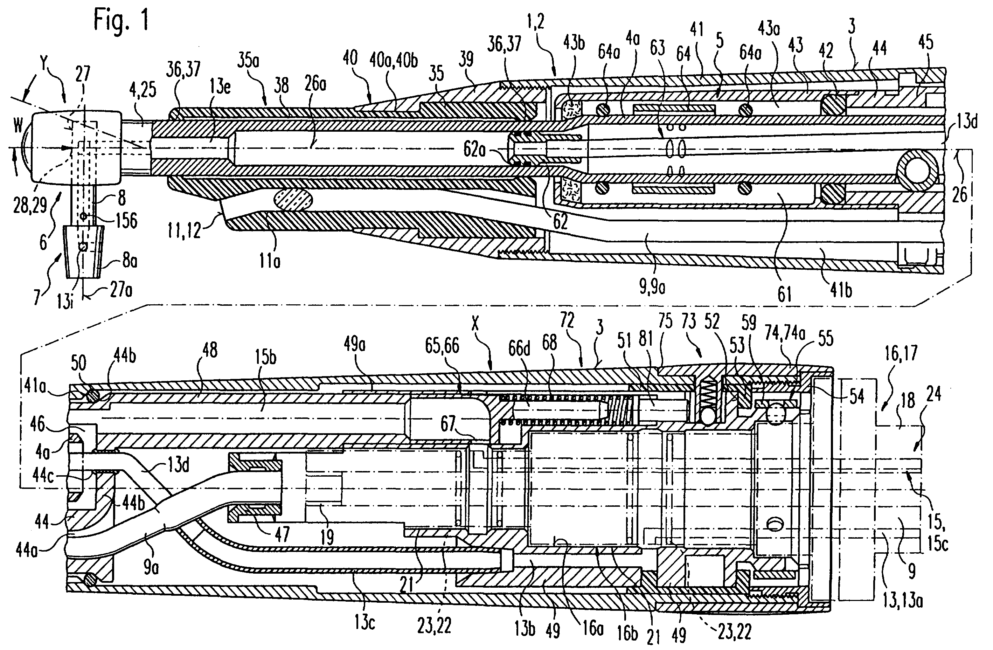

Der sich im Bereich des Handstücks 2 als Schwingstab 4a erstreckende Handstückschaft 25 ist gegen elastische Rückstellkräfte radial und vorzugsweise auch axial nachgiebig bzw. beweglich im Handstück 2 gelagert. Hierzu dient im vorderen Endbereich des Handstücks 2 eine Lagerhülse 35 aus elastischem Material, z.B. Kunststoff oder Gummi, in deren Durchgangsloch 36 der Schwingstab 4a mit umfangsseitiger Berührung aufgenommen ist, so daß er durch die Lagerhülse 35 zentriert ist. Bei der vorliegenden Ausgestaltung weist die Lagerhülse 35 in ihrem vorderen und hinteren Endbereich radial nach innen vorspringende, vorzugsweise im Querschnitt gerundete Lagerwülste 37 auf, zwischen denen ein den Schwingstab 4a umgebender Ringspalt 38 angeordnet ist. Die Lagerhülse 35 kann in einer Hülsenkappe 39 fest angeordnet sein, die mit einem Hülsengehäuse 41 fest verbunden, z.B. darin eingeschraubt ist, wie es Fig. 1 zeigt, und die umfangsseitig miteinander stufenlos abschließen und die Griffhülse 3 bilden.

Die Lagerhülse 35 überragt das Hülsengehäuse 41 bzw. die Hülsenkappe 39 und bildet damit einen Schutzmantel 35a für den Handstückschaft 25, der sich bis zur oder bis in die Nähe der Haltevorrichtung 6 bzw. ein noch zu beschreibendes Betätigungsglied 92a erstreckt. Zur axialen Fixierung der Lagerhülse 35 und oder des Schutzmantels 35a ist eine formschlüssige Verbindung 40 mit einer Ringnut 40a und einem darin einfassenden Ringzapfen 40b vorgesehen, der aufgrund des elastisch verformbaren Materials beim Einstecken selbsttätig einrastet. Durch den vorragenden elastischen oder weichelastischen Schutzmantel 35a werden bei der Behandlung aus der Vibration resultierende Beeinträchtigungen bei der Berührung des Werkzeugschaftes mit benachbarten Körperteilen, z.B. Zähnen, vermieden. Im Rahmen der Erfindung kann der Schutzmantel auch als separates Bauteil nur auf dem Werkzeugschaft 25 angeordnet und fixiert sein, z. B. darauf elastisch klemmend aufgeschoben oder mittels einer entsprechenden formschlüssigen Verbindung 40 darauf fixiert sein.

Im hinteren Bereich des Schwingstabs 4a ist die radial und axial elastisch nachgiebige Lagerung für den Schwingstab 4a durch einen Lagerring 42 aus elastisch verformbaren Material wie Kunststoff oder Gummi, insbesondere einen O-Ring, gebildet, in dem der Schwingstab 4a mit außenseitigem Kontakt sitzt, wobei der Lagerring 42 in einer den Schwingstab 4a mit radialem Ringraum 43a umgebenden Hülse 43 oder in einen sich von letzterer nach hinten erstreckenden Lagerteil 44 aufgenommen und gelagert ist, die bzw. das im Hülsengehäuse 41 radial und axial unbeweglich oder vorzugsweise gegen eine Rückstellkraft beweglich gelagert ist, insbesondere von hinten eingeschoben ist und mit einer vorderseitigen Schulterfläche an einer rückseitigen Schulterfläche des Hülsengehäuses 41 anliegt. Das Lagerteil 44 weist einen vorderseitigen Hülsenansatz 45 auf, der den Schwingstab 4a mit radialem Abstand umgibt, wobei das hintere Ende des Schwingstabs 4a in einem Freiraum 46 des Lagerteils 44 endet. Der vordere Hülsenansatz 45 ist in einander überlappender Anordnung mit der Hülse 43 verbunden, vorzugsweise schließend auf- oder eingesteckt und durch eine Dichtung abgedichtet, die hier durch den Lagerring 42 gebildet ist.

Der Schwingstab 4a kann mit den zugehörigen Lagerelementen koaxial zur Längsmittelachse 26 angeordnet sein. Bei der vorliegenden Ausgestaltung ist die Längsmittelachse 26a des Schwingstabes 4a bezgl. der Längsmittelachse des vorderen Endbereichs der Griffhülse 3 und der Lagerhülse 35 zur dem seitlich angeordneten Werkzeugkörper 8a abgewandten Seite hin versetzt und somit exzentrisch angeordnet, wobei die Ausmündungsöffnung 11 und der zugehörige Durchgangskanal 11a, in dem der Lichtleiter 9a aufgenommen ist, bezgl. des Schwingstabs 4a zur dem Werkzeug zugewandten Seite hin angeordnet ist, hier in der entsprechend exzentrischen Lagerhülse 35. Das rückseitige Ende des Lichtleiters 9a ist mittels einer Lagerhülse 47 in einer Verlängerung der Kupplungsausnehmung 16a angeordnet, wobei sich der Lichtleiter 9a durch einen exzentrischen Durchgangskanal 44a im Lagerteil 44 als seitlich bogenförmig ausgeformtes Teil nach vorne erstreckt. Dem Lichtleiter 9a gegenüberliegend weist das Lagerteil 44 einen exzentrischen rückseitigen Rohrstutzen 48 auf, an das sich rückseitig ein Hülsenvorsprung 49a einer Kupplungshülse 49 anschließt, die allein oder mit dem Lagerteil 44 als zusammengesetztes Bauteil radial allseitig und axial gegen eine Rückstellkraft beweglich im Hülsengehäuse 41 gelagert ist. Hierzu dient wenigstens ein die Kupplungshülse 49 umgebender Lagerring 51 aus elastisch verformbarem Material, der die Kupplungshülse 49 aufgrund seiner elastischen Rückstellkraft zentriert und radial elastisch nachgiebig lagert. Vorzugsweise weist der Lagerring 51 einen eine rückseitige Schulterfläche 52 hintergreifenden Innenflansch 53 auf, der die Kupplungshülse 49 auch bezgl. axialen nach hinten gerichteten Bewegungen elastisch nachgiebig lagert. Bei der vorliegenden Ausgestaltung ist die durch die Kupplungshülse 49 und das Lagerteil 44 gebildete Baueinheit zwischen dem Innenflansch 53 und dem Lagerring 42 in beide axiale Richtungen elastisch nachgiebig gelagert. Zusätzlich kann zwischen zugehörigen Schulterflächen 41a, 44b, hier zwischen einer Innenschulter des Hülsengehäuses 41 und einer Außenschulter des Lagerteils 44, ein axial und vorzugsweise auch diametral wirksamer Lagerring 50 aus elastisch nachgiebigem Material angeordnet sein, wobei aufgrund der elastischen Rückstellkraft der elastisch nachgiebigen Lagerelemente eine Mittenzentrierung gewährleistet ist und zwar sowohl axial als auch diametral. Der Lagerring 51 ist in axialer Richtung formschlüssig fixiert, z.B. dadurch, daß sein Innenflansch 53 an einer nach vorne gerichteten Schulterfläche einer in das hintere Ende des Hülsengehäuses 41 eingeschraubte Gewindehülse 54 anliegt.

Im hinteren Bereich der Steck-Drehkupplung 16 ist eine manuell überdrückbare Verrastungsvorrichtung 55 zum überdrückbaren Verrasten in der Kupplungsendstellung vorgesehen. Bei der vorliegenden Ausgestaltung weist die Verrastungsvorrichtung 55 eine oder mehrere auf den Umfang in radialen Löchern der Kupplungshülse 49 verteilt angeordnete, vorzugsweise durch Kugeln gebildete Verrastungselemente, gebildet, die soweit in die Löcher eintauchen können, daß die radial inneren Enden der Verrastungselemente in eine Ringnut im Kupplungszapfen 16b einrasten können. Beim manuellen Überdrücken der Steck-Drehkupplung werden die Verrastungselemente selbsttätig gegen eine sich nach innen radial beaufschlagende Feder in Form eines Federrings 59 aus der Ringnut herausgedrückt. Die Löcher sind an ihren inneren Enden verjüngt, so daß das oder die Verrastungselemente, vorzugsweise Kugeln bei entferntem Kupplungszapfen 16b nicht nach innen herausfallen können.

Die Medienleitungen erstrecken sich im Anschlußstück 17 als z.B. achsparallele Kanäle 13a, 15a, die sich in der zugehörigen Durchgangs-Querebene in Form von Winkelkanälen im Kupplungszapfen 16b und in der Kupplungshülse 49 fortsetzen, wobei die eine Durchgangs-Querebene E1 bezüglich der anderen Durchgangs-Querebene E2 der Druckluftleitung axial versetzt ist und die Winkelkanäle in der Kupplungshülse 49 mit 13b, 15b bezeichnet sind. An den Winkelkanal 13b der Leitung 13 für Behandlungsflüssigkeit schließt sich ein Rohr oder Schlauch 13c an, der stromab mit einem weiteren Rohr oder Schlauch 13d verbunden ist, der eine Rückwand 44b des Lagerteils 44 in einer Durchführungshülse 44c abgedichtet durchsetzt und sich im Hohlraum 61 des hülsenförmigen Schwingstabs 4a bis in dessen mittleren Bereich erstreckt, in dem eine Endhülse 62 mittels einem oder zwei Dichtungsringen 62a abgedichtet im hülsenförmigen Schwingstab 4a sitzt und dicht mit dem Rohr 13d verbunden ist, insbesondere darauf aufgesetzt ist.

Dem Schwingungserreger 5 ist eine Steuervorrichtung zum Verringern oder Vergrößern seiner Leistung zugeordnet. Hierdurch läßt sich seine Leistungsfähigkeit bzw. die Intensität der Vibration bzw. Schwingungen und die Größe der Amplituden wahlweise verringern oder vergrößern und somit einstellen. Dabei kann ein im einzelnen noch zu beschreibender Leistungsregler 71 zum automatischen Regeln einer vorzugsweise konstanten Leistung und/oder eine vorzugsweise manuell einstellbare Steuervorrichtung 72 vorgesehen sein, mit dem bzw. mit der die Leistung oder die dem Schwingungserreger 5 zuführbare Antriebsenergiemenge veränderlich und verringerbar und vergrößerbar ist und selbsttätig oder durch eine manuelle Einstellvorrichtung 73 in Stufen oder stufenlos einstellbar ist. Hierdurch kann die Leistung des Behandlungsinstruments an die zu verrichtende Arbeit, z.B. Grob- und Feinarbeit oder Grob-, Mittel- und Feinarbeit oder an unterschiedliche Arten der Behandlung und/oder an hinsichtlich Form und/oder Größe und/oder grober und feiner oder grober, mittlerer und/oder feiner Wirksamkeit unterschiedliche Werkzeuge 7 angepaßt werden.

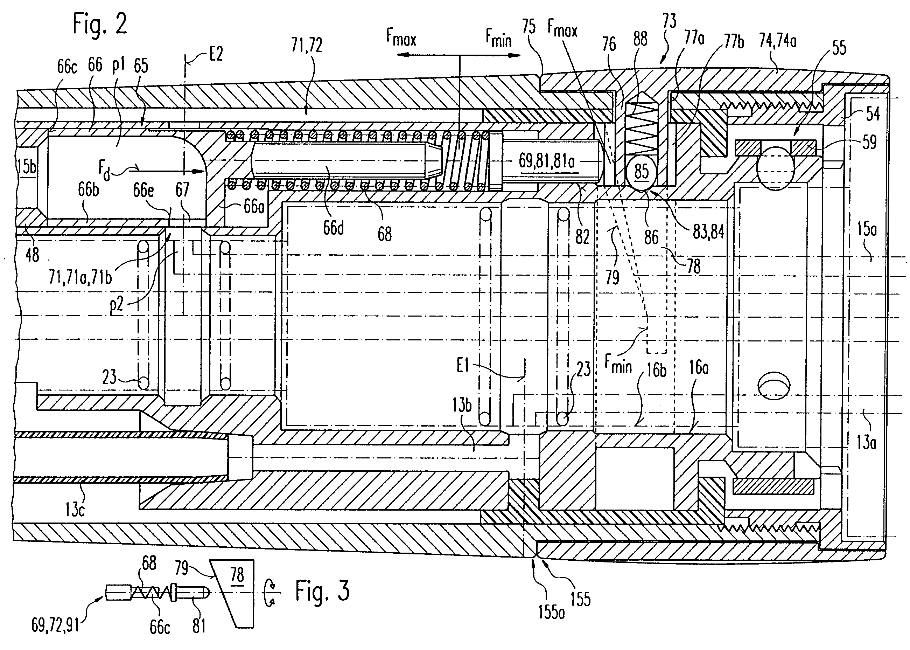

Die manuell betätigbare Einstellvorrichtung 73 weist ein Einstellglied 74 auf, das von außen manuell zugänglich und axial oder in Umfangsrichtung verstellbar ist und in Antriebsverbindung mit der Steuervorrichtung 72 steht oder eine Steuervorrichtung bildet. Bei der vorliegenden Ausgestaltung ist das Einstellglied 74 eine Einstellhülse 74a die das Handstück 2 vorzugsweise in dessen hinteren Bereich umgibt und insbesondere in einer eine Ringführung bildenden Ringausnehmung 75 versenkt angeordnet und axial oder vorzugsweise in Umfangsrichtung verstellbar ist. Bei der vorliegenden Ausgestaltung ist die Ringausnehmung 75 rückseitig durch eine Schulterfläche der Einschraubhülse 54 begrenzt. Von der Innenmantelfläche der Einstellhülse 74a ragt ein Tragsteg 76 radial einwärts vor, der sich über einen Teil des Umfangs erstreckt und Schlitze 77a, 77b im Hülsengehäuse 41 und den davon radial einwärts angeordneten Bauteilen, hier in der Kupplungshülse 49 und im Lagerring 51, mit Bewegungsspiel durchfaßt und an seinem inneren Ende ein sich über einen Abschnitt des Umfangs erstreckendes Segment 78 trägt, das an einer Seite eine Schräg- oder Kurvenfläche 79 aufweist, die mit einem Verstellglied 81 zusammenwirkt, das mit der Steuervorrichtung 72 in Wirkverbindung steht, wie es Fig.3 schematisch und Fig.1 und 2 als Ausführungsbeispiel zeigen. Die Schräg- oder Kurvenfläche 79 kann vorderseitig und rückseitig am Segment 78 angeordnet sein. Bei der vorliegenden Ausgestaltung, bei der das Einstellglied 74 im hinteren Endbereich des Handstücks 2 befindet, ist die Schräg- oder Kurvenfläche 79 vorderseitig angeordnet, und das Verstellglied 81 ist ein achsparallel in einer Führung 82 verschiebbar gelagerter Verstellstift 81a, der durch die Kraft einer Feder, hier einer Druckfeder gegen die Schräg- oder Kurvenfläche 79 beaufschlagt ist und diese somit bei einer Hin- und Herverdrehung des Verstellglieds 74 abzugreifen vermag. Die Führung 82 kann durch eine axparallele Bohrung in der drehgesichert gelagerten Kupplungshülse 49 gebildet sein.

Die Einstellvorrichtung 73 kann in Stufen oder stufenlos einstellbar sein. In beiden Fällen ist es vorteilhaft, der Einstellvorrichtung 73 eine Feststellvorrichtung 83 zuzuordnen, die ein Feststellen der Einstellvorrichtung 73 in der jeweils eingestellten Position ermöglicht und dadurch eine unbeabsichtigte Verstellung verhindert. Hierbei kann es sich um eine Bremsvorrichtung handeln, die aufgrund ihrer Schwergängigkeit eine unbeabsichtigte Verstellung des Verstellglieds 74 verhindert. Bei der vorliegenden Ausgestaltung ist eine überdrückbare Verrastungsvorrichtung 84 mit einem vorzugsweise durch eine Kugel gebildeten, radial beweglich geführten Verrastungselement 85 vorgesehen, das beim Drehen der Einstellhülse 74a in Kalotten 86 manuell überdrückbar ein- und ausrastbar ist, die auf einer Außenmantelfläche der Kupplungshülse 49 in Umfangsrichtung hintereinaneinanderliegend angeordnet sind, hier im Grund der Nut bzw. des Schlitzes 77b. Das Verrastungselement 85 kann in einer radialen Bohrung im Tragsteg 76 radial verschiebbar gelagert sein und durch eine in der Bohrung angeordnete Druckfeder 88 gegen die Kalotten 86 vorgespannt sein. Das Verstellglied 81 wirkt auf den in seiner Leistungsfähigkeit einstellbaren Schwingungserreger 5, so daß dessen Leistung mit dem Einstellglied 74 wahlweise zu vergrößern oder zu verringern ist.

Beim vorliegenden Ausführungsbeispiel, bei dem der Schwingungserreger 5 pneumatisch antreibbar bzw. erregbar ist, kann die Steuervorrichtung 72 ein Steuerventil 65 aufweisen zwecks Steuerung des am Schwingungserreger 5 wirksamen Druckes p1. Hierbei kann das Einstellglied 74 mittelbar oder unmittelbar mit einem Ventilschieber 66 verbunden sein, der die Größe einer Ventilöffnung 67 in Abhängigkeit von der Einstellung des Einstellglieds 74 steuert. Die Ventilöffnung 67 befindet sich in der Zuführungsleitung 15, hier stromab vom Kupplungszapfen 16b im Bereich des Winkelkanals 15b. Zur Verringerung der Schwingungsleistung wird der Ventilschieber 66 mit der Einstellvorrichtung 73 im Sinne einer Verkleinerung der Ventilöffnung 67 verschoben, beim vorliegenden Ausführungsbeispiel nach vorne, so daß die Ventilöffnung 67 den am Schwingungserreger 5 anstehenden Druck p1 im Sinne einer verstellbaren Drossel verringert. Zur Vergrößerung der Leistung wird die Ventilöffnung 67 im umgekehrten Sinne vergrößert, wodurch ein größerer anstehender Druck p1 eingestellt wird. Die Rückihrung des Einstellglieds 74 kann durch eine Rückholfeder erfolgen, die das Einstellglied 74 gegen die Schräg- oder Kurvenfläche 79 vorspannt.

Es ist vorteilhaft, zusätzlich oder anstelle der Steuervorrichtung 72 zur Einstellung des anstehenden Druckes p1 eine automatische Druck-Regelvorrichtung 71a mit einem Druckregelventil 71b vorzusehen, die bzw. das unabhängig vom vorhandenen Betriebsdruck p2 im Zuführungsleitungsabschnitt 15a einen im wesentlichen konstanten wirksamen Druck p1 einstellt. Hierdurch werden auch bei beträchtlichen Toleranzen des wirksamen Druckes p1 im wesentlichen gleiche Arbeitsbedingungen und eine etwa vergleichbare Leistung bzw. Intensität des Werkzeugs 7 beim Anschluß des Behandlungsinstruments an Versorgungsleitungen 18 und Versorgungseinrichtungen mit unterschiedlichen Betriebsdrücken p2, insbesondere von unterschiedlichen Herstellern, erreicht.

Sowohl beim Vorhandensein eines Steuerventils 65 als auch eines Regelventils 71b ist der Ventilschieber 66 in der Zuführungsleitung 15, hier im achsparallelen Zuführungsleitungsabschnitt 15b, angeordnet, vorzugsweise darin längsverschiebbar gelagert, wobei die Ventilköffnung 67 durch ein Stirnfläche des Ventilschiebers 66 gesteuert werden kann. Der Ventilschieber 66 kann auch eine topfförmige Form aufweisen, wobei die Ventilöffnung 67 in der Umfangswand der Topfform angeordnet sein kann.

Beim vorliegenden Ausführungsbeispiel gemäß Fig. 1 bis 3 sind eine Steuervorrichtung 72 oder eine manuelle Einstellvorrichtung 73 in Kombination mit einem Druckregelventil 71b vorgesehen. Der Ventilschieber 66 wird auf seiner einen Stirnseite vom wirksamen Druck p1 beaufschlagt und auf seiner anderen Stirnseite mittels einer Druckfeder 68 entgegengesetzt in seine Offenstellung vorgespannt. Bei der vorliegenden Ausgestaltung ist der Ventilschieber 66 eine runde oder unrunde topfförmige Hülse mit einer Bodenwand 66a an seinem dem Schwingungserreger 5 abgewandten Ende. Die Ventilöffnung 67 ist in der radial innenliegenden Umfangswand 66b mit dem radialen Abschnitt des Zuführungsleitungsabschnitts 15b zusammenwirkend angeordnet. In der Offenstellung ist der Ventilschieber 66 durch die Feder 68 gegen einen Anschlag 66c gespannt, hier den Rohrstutzen 48. Vom Ventilschieber 66 erstreckt sich ein Federdorn 66d nach hinten, auf dem die Druckfeder 68 sitzt und gegen ein rückseitiges Widerlager 69, hier gegen das Verstellglied 81, abgestützt ist.

Im Funktionsbetrieb strömt die Druckluft vom Zuführungsleitungsabschnitt 15b durch den am rückseitigen Ende vorhandenen Ringspalt in den Hohlraum 61 des hülsenförmigen Schwingstabs 4a, der im mittleren Bereich und vor der Endhülse 62 ein oder mehrere radiale und/oder sekantiale Durchströmlöcher 63 aufweist, in deren Bereich der Schwingstab 4a mit radialem Bewegungsspiel von einer Taumel- oder Vibrationshülse 64 umgeben ist, die mit axialem Bewegungsspiel zwischen zwei Anschlägen 64a angeordnet ist, die jeweils durch einen Ring aus elastischem Material, z.B. einen O-Ring, gebildet sein können, der in einer Ringnut im Schwingstab 4a sitzt. Während der Durchströmung des Spalts zwischen dem Schwingstab 4a und der Vibrationshülse 64 wird letztere in Schwingung versetzt, die sie durch mechanisches Anschlagen auf den Schwingstab überträgt. Der Spalt ist so groß bemessen, daß die Vibrationshülse 64 nicht gegen die Innenwandung der sie umgebenden Hülse 43 schlägt. Aus dem Ringraum 43a strömt die Luft bzw. Abluft durch einen zwischen dem Schwingstab 4a und dem vorderen Endbereich der Hülse 43 angeordneten luftdurchlässigen Stützring 43b, der perforiert sein kann und aus elastischem oder nachgiebigem Material wie Kunststoff oder luftdurchlässigem Material wie z.B. Filz bestehen kann, in den freien Hohlraum 41b des Hülsengehäuses 41 und durch eine geeignete insbesondere hintere Öffnung (nicht dargestellt) im Hülsengehäuse 41 ins Freie oder z.B. durch den Kanal 44a und durch einen geeigneten Abführungskanal im Kupplungszapfen 16b zurück ins Freie. Der Stützring 43b ist axial fixiert, hier zwischen einer gegebenenfalls kegelförmigen Schulterfläche am Schwingstab 4a und einem Innenflansch der Hülse 43.

Die Hülse 43 bildet in Verbindung mit dem Stützring 43b ein Innengehäuse bzw. eine Kapselung des Schwingungserregers 5, wodurch eine wesentliche Geräuschminderung auf einfache und kostengünstige Weise erreicht wird.

Die Ventilöffnung 67 ist eine verstellbare Drossel, die einen Druckabfall erzeugt, der den wirksamen Druck p1 bestimmt. Wenn der Betriebsdruck p2 größer oder kleiner als ein bestimmter Wert ist, ändert sich zunächst auch der Druck p1, der die den Ventilschieber 66 gegen die Feder 68 beaufschlagende Druckkraft Fd erzeugt, wobei die Stellung des die Größe der Ventilöffnung 67 bestimmenden Öffnungsrandes 66e und somit auch die Größe der Drossel, die den wirksamen Druck p1 erzeugt, durch das Gleichgewicht der Federkraft und der Druckkraft Fd bestimmt ist. Im Funktionsbetrieb nimmt der Ventilschieber 66 eine von der dargestellten Anschlagstellung (Außerfunktionsstellung) entfernte Funktionsstellung ein, in der z.B. bei einem mittleren Betriebsdruck p2 die Ventilöffnung 67 eine Größe im mittleren Bereich einnimmt. Bei einem niedrigeren Betriebsdruck p2 wird der Ventilschieber 66 in eine Gleichgewichtsstellung (hier nach links) verschoben, in der er eine größere Ventilöffnung 67 steuert. Bei einem vergrößerten Betriebsdruck p2 nimmt der Ventilschieber 66 eine (hier nach rechts verschobene) Gleichgewichtsstellung ein, in der er eine kleinere Ventilöffnung 67 steuert. Aufgrund dieser Funktion regelt das Regelventil 71b bei in einem bestimmbaren Druckbereich unterschiedlichen Betriebsdrücken automatisch einen im wesentlichen konstanten anstehenden Druck p1. Diese Druckregelung funktioniert sowohl dann, wenn eine Einstellvorrichtung 73 oder eine sonstige Steuervorrichtung 72 fehlt, als auch dann, wenn - wie beim vorliegenden Ausführungsbeispiel - der Druck- bzw. Leistungsregler 71 in Kombination mit der Einstellvorrichtung 73 oder einer sonstigen Steuervorrichtung 72 angeordnet ist oder auch dann, wenn - wie beim vorliegenden Ausführungsbeispiel - die manuelle Einstellvorrichtung 73 oder eine sonstige Steuervorrichtung 72 auf das Druckregelventil 71b bzw. auf dessen Ventilschieber 66 einwirkt. Vorzugsweise ist eine gemeinsame Ventilöffnung 67 für das Druckregelventil 71b und die manuelle Einstellvorrichtung 73 oder eine sonstige Steuervorrichtung 72 vorhanden. Hierdurch wird der Bau- und Herstellungsaufwand beträchtlich verringert und die Bauweise vereinfacht.

Beim vorliegenden Ausführungsbeispiel wird mittels der Einstellvorrichtung 73 die Vorspannkraft der Ventilfeder 68 verändert und zwar für hohen Leistungsbedarf vergrößert und für niedrigeren Leistungsbedarf verringert. Bei höherem Leistungsbedarf wird somit das Verstellglied 81 in Richtung auf das Steuer- oder Regelventil 65, 71b verschoben und bei geringerem Leistungsbedarf vom Steuer- oder Regelventil 65, 71b entfernt d.h. nach hinten verschoben, was durch die Bezugszeichen Fmin und Fmax verdeutlicht ist. Beim vorliegenden Ausführungsbeispiel wird mittels der Einstellvorrichtung 73 die Leistung des Leistungsreglers 71 im Sinne einer Vergrößerung oder Verringerung der am Werkzeug 7 abgebbaren Leistung verändert und zwar unabhängig von der Größe des Betriebsdruckes p2. Bei einer im Rahmen der Erfindung möglichen Ausgestaltung ohne Regelventil 71b läßt sich die Leistung mittels der manuellen Einstellvorrichtung 73 oder einer sonstigen Steuervorrichtung 75 ebenfalls wahlweise verringern und vergrößern und zwar abhängig vom vorhandenen Betriebsdruck p2, wobei das Verstellglied 81 ebenfalls auf den Ventilschieber 66 des nunmehr eine Steuerfunktion ausführenden Steuerventils 65 wirkt. Hierzu kann das Verstellglied 81 direkt mit dem Ventilschieber 66 verbunden sein, z.B durch einen Federdorn 66d in einer erforderlichen Länge gebildet sein.

Hierdurch läßt sich das Behandlungsinstrument 1 nicht nur an unterschiedliche Behandlungsmethoden sondern auch an Werkzeuge 7 unterschiedlicher Form und/oder Größe und/oder abrasiver Abtragungsfähigkeit, z.B. grob und fein oder grob, mittel und fein, anpassen, wobei auch eine Abstimmung unter Berücksichtigung von Werkzeugen 7 unterschiedlicher Massen und/oder Formen möglich ist. Außerdem lassen sich mit der erfindungsgemäßen Einstellvorrichtung 73 einander störende Schwingungszustände durch Veränderung der Schwingungsamplituden weitgehend ausschalten.

Die Hauptrichtung der Schwingungs-Aplitude ist im wesentlichen quer zum Schwingteil 4a und somit im wesentlichen in der Längsrichtung des Werkzeugs 7 gerichtet. Aufgrund der radialen und axialen elastisch nachgiebigen Lagerung des Schwingteils 4a stellen sich jedoch unter anderem durch Resonanzen hervorgerufene räumlich Schwingungen ein, so daß das Werkzeug 7 auch in Querrichtung abrasiv wirksam ist.

Beim vorliegenden Ausführungsbeispiel weist der Oszillations- bzw. Schwingungsantrieb eine Frequenz im Schall- oder Ultraschallbereich von etwa 4 bis 8 kHz, vorzugsweise etwa 6 kHz, auf, wobei sich im Bereich des Werkzeugs 7 eine Amplitude der räumlichen Bewegungen von etwa 0,05 mm bis 0,2 mm, insbesondere etwa 0,1 mm ergibt.

Das erfindungsgemäße Behandlungsinstrument eignet sich deshalb besonders gut für unterschiedliche Werkzeuge 7, die dem Behandlungsinstrument als Werkzeug-Sortiment zugeordnet sind und sich aufgrund unterschiedlicher Form und/oder Größe und/oder Zweckbestimmung voneinander unterscheiden.

Die erfindungsgemäße Haltevorrichtung 6 ermöglicht nicht nur eine sichere Halterung des Werkzeugs 7 am Handstückschaft 25, sondern auch eine leichte, handhabungsfreundliche und schnelle Einspannung oder Lösung des Werkzeugs 7. Zum Lösen und Herausbewegen des Sicherungsteils 29 aus der Sicherungsausnehmung 28 ist ein Betätigungsglied 91 als unverlierbares Bauteil im vorderen Endbereich des Handstückschaftes 25 zwischen einer Bereitschaftsstellung und einer Lösestellung in einer Führung beweglich gelagert. Das Betätigungsglied 91 steht direkt oder indirekt mit dem Sicherungsteil 29 in Verbindung, wobei seine Bewegungsrichtung zwischen der Bereitschaftsstellung und der Lösestellung parallel zur Bewegungsrichtung des Sicherungsteils 29 verläuft, d.h. bezgl. des Werkzeugschaftes 8 radial gerichtet ist. Bei dem vorliegenden Ausführungsbeispiel ist das Betätigungsglied ein Betätigungsschieber 92, der seitlich und/oder von der dem Werkzeug 7 abgewandten Seite des Handstuckschaftes 25 her manuell zugänglich ist und vorzugsweise durch eine dem Werkzeugschaft 25 umgebende und darauf in dessen Längsrichtung verschiebbare Betätigungshülse aus Kunststoff oder vorzugsweise Metall gebildet ist, wobei die hohlzylindrische Mantelfläche des Handstückschafts 25 eine Längsführung 93 für den Betätigungsschieber 92 bildet. Gemäß Fig. 4 ist die Betätigungshülse 92a so lang bemessen, daß sie das Steckloch 27 beidseitig überragt, wobei sie in Flucht mit dem Steckloch 27 eine Steckausnehmung 94 auf ihrer dem Werkzeug 7 zugewandten Seite aufweist, die unter Berücksichtigung des Verstellweges der Betätigungshülse 92a durch ein Langloch gebildet ist, das zur Rückseite der Betätigungshülse 92a hin in Form eines Schlitzes auslaufen kann. Bezüglich der Steckausnehmung 94 nach vorne versetzt weist die Betätigungshülse 92a zwei bezüglich der Längsmittelachse 27a des im Handstückschaft 25 als Durchgangsloch ausgebildeten Stecklochs 27 ein Durchgangsloch 95 auf, in dem ein Querstift 96 vorzugsweise mit endseitig auf ihn eingesetzten Kappen 97 gelagert ist, wobei der Querstift 96 bzw. die Kappen 97 einen vorzugsweise nach vorne auslaufenden Schlitz 98 im vorderen Endbereich des Handstückschaftes 25 mit Bewegungsspiel durchfassen. Hierdurch ist eine Drehsicherung für die Betätigungshülse 92a geschaffen. Auf dem mittleren Bereich des Querstifts 96 sitzt das Sicherungsteil 29 mit einem entsprechenden Querloch 99, das beim vorliegenden Ausführungsbeispiel für die Kappen 97 stufenförmig ausgebildet ist. Das Sicherungsteil 29 befindet sich in einem nach vorne auslaufenden koaxialen Aufnahmeloch 101 im Handstückschaft 25, in dessen vorderen Bereich ein Innengewinde angeordnet ist. Das Sicherungsteil 29 weist an seiner Rückseite eine Sicherungsnase 29a in Form einer gerundeten Kuppel auf, deren Flanken so steil sein können, daß ein Überdrücken der so gebildeten Verrastungsvorrichtung 102 durch axialen Zug am Werkzeug 7 nicht möglich ist oder so flach verlaufen können, daß ein selbsttätiges Verdrängen der Sicherungsnase 29a aus der Sicherungsnehmung 28 durch ein Ziehen am Werkzeug 7 möglich ist. Vorderseitig ist das Sicherungsteil 29 mit einem Ringwulst als Federteller 103 ausgebildet, wobei eine Druckfeder 104 zwischen dem Federteller 103 und einem Federteller 105 an einem in das Aufnahmeloch 101 eingeschraubten Schraubdeckel 106 angeordnet ist. Der Schraubdeckel 106 kann vorderseitig durch eine vorzugsweise konvex gerundete Kappe 107 vorzugsweise aus Kunststoff abgedeckt sein, die durch einen in ein Verrastungsloch 108 einfassenden Verrastungsstift 109 am Schraubdeckel 106 verrastet ist. Letzterer weist zum Ein- und Ausschrauben ein Werkzeugangriffselement auf, hier zwei achsparallele Sacklöcher 111, in die ein Werkzeug mit entsprechenden Mitnehmerzapfen einführbar ist. Zur Abdichtung der Längsführung 93 sind zu beiden Seiten des Stecklochs 27 Dichtungsringe 112 vorzugsweise in Form O-Ringen vorgesehen, die in Ringnuten angeordnet sind, die sich in der Außenmantelfläche des Handstückschafts 25 oder in der Innenmantelfläche der Betätigungshülse 92a befinden. Die rückseitige Grundfläche der Schlitze 98 bildet einen Anschlag 98a für die nach hinten gerichtete Bewegung des Sicherungsteils 29, in der die Sicherungsnase 29a in das Steckloch 27 hineinragt. Die bereits soweit beschriebene Verrastungsvorrichtung 102 für den Werkzeugschaft 8 ist funktionsfähig und aus Gründen einer vorteilhaften und einfachen Handhabung sowie sicheren Halterung vorteilhaft. Beim Einstecken des Werkzeugschafts 8 in das Steckloch 27 wird die Verrastungs- oder Sicherungsnase 29a mittels einer vorzugsweise auch am freien Ende des Werkzeugschaftes 8 vorhandenen Einführungsschräge 113 oder -rundung in seine Freigabestellung verdrängt, so daß der Werkzeugschaft 8 weiter eingeschoben werden kann bis sein freies Ende an der Innenwandung der Betätigungshülse 92a anschlägt. In dieser Stellung federt die Sicherungsnase 29a selbsttätig in die zugehörige Sicherungsausnehmung 28 ein, wobei vorzugsweise die Flächen der Sicherungsausnehmung 28 den Bewegungsanschlag in der Sicherungsstellung bilden.

Wenn die Sicherungsausnehmung 28 durch eine Ringnut gebildet ist, ist eine Halterung für den Werkzeugschaft 8 gegeben, bei der ein Drehen des Werkzeugs in der Haltevorrichtung 6 möglich ist, wobei auch eine ungebremste, d.h. freie Drehbarkeit dann gewährleistet ist, wenn der Anschlag 98a so angeordnet ist, daß die Sicherungsnase 29a zwar in die Ringnut einfaßt, jedoch nicht oder nur leicht gegen den Ringnutgrund drückt.

Bei der vorliegenden Ausgestaltung mit mehreren auf dem Umfang verteilt angeordneten Kalotten 31 ist eine Drehposition-Einstellvorrichtung 114 gegeben, die unterschiedliche Drehstellungen des Werkzeugs 7 dadurch ermöglicht, daß die Verrastung in der jeweils zugehörigen Kalotte 31 eine Drehsicherung bildet.

Dabei ist es möglich, diese Drehposition-Einstellvorrichtung 114 so auszubilden, daß bei Überschreitung eines bestimmten Drehmoments die Verrastungsvorrichtung 110 selbsttätig überdrückt wird und die Sicherungsnase 29a selbsttätig aus der zugehörigen Kalotte 31 herausgedrückt wird. Hierdurch bedarf es lediglich einer Anpassung der Flanke 29b der Sicherungsnase 29a und/oder der Kegelflächen der Kalotten 31 an einen bestimmten Winkel, bei dem unter Berücksichtigung des bestimmten Drehmomentes die Lösung der Verrastungsvorrichtung 110 selbsttätig erfolgen soll.

Zum willkürlichen Lösen der Verrastungsvorrichtung 102, z.B. wenn ein Werkzeug 7 gelöst oder ausgetauscht werden soll, wird das Betätigungsglied 91 manuell durch Fingerdruck in Richtung des Pfeiles 115 gegen die Kraft der Feder 104 nach vorne verschoben, wobei die Verrastungsvorrichtung 102 gelöst wird und der Werkzeugschaft 8 aus der Haltevorrichtung 6 herausgezogen werden kann.

Bei einer vorbeschriebenen Verrastungsvorrichtung ermöglicht eine axiale Weiterführung der Leitung 13 als Kanal 13e im Handstückschaft 25 bis zum Steckloch 27 und im Werkzeugschaft 8 vorhandene daran anschließende radiale und axiale Kanalabschnitte 13f und 13g die Zuführung der Behandlungsflüssigkeit zur Behandlungsstelle, wobei die Flüssigkeit bei nicht dargestellten Ausmündungsöffnungen im Bereich der abrasiven Arbeitsflächen des Werkzeugs 7 ausmünden können. Alternativ oder zusätzlich kann auch eine sich vom Querbereich der Sicherungsausnehmung 28 zum Werkzeugkörper 8a hin erstreckende Längsnut 13h in der Mantelfläche des Werkzeugschafts 8 vorgesehen sein, durch die Behandlungsmittel außenseitig am Werkzeugkörper 8a zur Behandlungstelle rinnen kann.

Es ist im weiteren vorteilhaft, die Haltevorrichtung 6 mit einer Vorrichtung 116 zum Ausdrücken des Werkzeugschaftes 8 aus der Haltevorrichtung 6 auszubilden. Eine solche Vorrichtung 116 kann mit einem Keiltrieb und einer Keilfläche 117 oder Kurvenfläche gebildet sein, die beim Bewegen des Betätigungsgliedes 91 in seine Lösestellung gegen den Werkzeugschaft drückt und ihn in seine Entnahmerichtung verschiebt. Bei der vorliegenden Ausgestaltung ist die Keilfläche 117 durch die rückseitige Flanke einer vorzugsweise kegelförmigen Kalotte 118 in der Innenmantelfläche der Betätigungshülse 92a gebildet, wobei die Keilfläche 117 so angeordnet ist, daß in der Bereitschaftsstellung der Betätigungshülse 92a gem. Fig. 4 der Werkzeugschaft 8 an einem tiefen Punkt der Keilfläche 117 anliegt oder davon einen geringen Abstand aufweist. Das Stirnende des Werkzeugschaftes 8 ist vorzugsweise kugel- oder kegelförmig gerundet, damit die Keilfläche 117 im mittleren Bereich der Stirnfläche angreift. Bei der vorliegenden Ausgestaltung ist am freien Ende des Werkzeugschaftes 8 ein kuppelförmiges Druckelement oder vorzugsweise eine Kugel 119 in eine endseitige Ausnehmung 121 teilweise versenkt angeordnet und darin gegen Herausfallen gesichert. Hierdurch ist eine im wesentlichen zentraler Angriffspunkt für die Keilfläche 117 am Werkzeugschaft 8 gegeben. Wenn die Kugel 119 frei drehbar in der Ausnehmung 121 gehalten ist, werden die Reibungskräfte beim Ausstoßen des Werkseugschafts 8 wesentlich verringert und dadurch die Handhabung erleichtert.

Da dem Sicherungsteil 29 ein Betätigungsglied 91 zum Entrasten zugeordnet ist, können die Flanken 29b zwecks Erzielung größerer axialer Sicherungskräfte so steil oder auch rechtwinklig zur Schaftachse 27a ausgebildet sein, daß kein Überdrücken bzw. Verdrängen des Sicherungsteils 29 aus der Sicherungsausnehmung 28 möglich ist.

Zusätzlich zur Verrastungsvorrichtung 102 kann ihr vorzugsweise gegenüberliegend, bei der vorliegenden Ausgestaltung rückseitig vom Steckloch 27, eine Verrastungsvorrichtung 125 vorgesehen sein mit einer ebenfalls kuppelförmig gerundeten Sicherungsnase 125a an einem Sicherungsteil 125b, das zwischen einer in die Sicherungsausnehmung 28 einfassenden Sicherungsstellung und einer sie freigebenden Freigabestellung verschiebbar und durch die Kraft einer Feder, hier einer Druckfeder 126, in seine Sicherungsstellung vorgespannt ist, so daß die Sicherungsnase 125a beim Einstecken des Werkzeugschaftes 8 selbsttätig einrastet. Bei dieser Ausgestaltung ist jedoch keine direkte mechanische Verbindung zwischen dem Betätigungglied 91 und dem Sicherungsteil 125b vorgesehen. Deshalb sind bei dieser Verrastungsvorrichtung 125 die Flanken 29b der Sicherungsnase 125a oder der Sicherungsausnehmung 28 bzw. Kalotten 31 so flach auszubilden, so daß die Sicherungsnase 125a bei einer Verschiebung des Werkzeugschafts 8 im Steckloch 27 selbsttätig aus der Sicherungsausnehmung 28 verdrängt wird und diese Verrastungsvorrichtung 125 somit manuell überdrückbar ist.

Im Rahmen der Erfindung ist es möglich, die Haltevorrichtung 6 nur mit der Verrastungsvorrichtung 125 auszubilden, d.h. ohne die Verrastungsvorrichtung 102 und auch ohne das Betätigungsglied 91. Bei einer solchen Ausgestaltung rastet die Sicherungsnase 125a beim Einstecken des Werkzeugschafts 8 selbsttätig ein, und die Verrastungsvorrichtung 125 läßt sich durch die Ausübung einer axialen Zugkraft am Werkzeug 7 selbsttätig lösen, wobei die Sicherungsnase 125a aus der Sicherungsausnehmung 28 verdrängt wird. Bei entferntem Werkzeug 7 ist die Sicherungsnase 125a oder das Sicherungsteil 125b formschlüssig daran gehindert, in das Steckloch 27 hineinzufallen.

Bei der vorliegenden Ausgestaltung ist die Sicherungsnase 125a an einem zylindrischen Stift 125c mit einem rückseitigen Flansch 125d ausgebildet, der in einer Führungsbuchse 127 zwischen seiner Sicherungsstellung und seiner Freigabestellung quer zum Werkzeugschaft 8 verschiebbar gelagert ist. In der Führungsbuchse 127 ist die den Stift 125c gegen den Werkzeugschaft 8 vorspannende Druckfeder 126 angeordnet, die sich rückseitig an einer Schulterfläche abstützt. Die Führungsbuchse 127 ist von vorne in ein entsprechendes Gewindeloch 128 eingesetzt und darin verschraubt. Am vorderen Ende der Führungsbuchse 127 ist eine nach hinten gerichtete Schulterfläche 129 vorgesehen, die einen Anschlag 131 bildet, der den Stift 125c am Herausfallen hindert. Im übrigen kann diese Verrastungsvorrichtung 125 entsprechend der Verrastungsvorrichtung 102 ausgebildet sein. Für den Durchgang der Medienleitung 13 weist der Stift 125c einen axialen Durchgangskanal 13i auf.

In Fig. 5 ist ein weiteres Ausführungsbespiel dargestellt, bei dem der die Griffhülse bzw. das Hülsengehäuse 41 überragende Handstückschaft 25 als separates Bauteil ausgebildet ist, das durch eine lösbare Verbindung, vorzugsweise eine Schraubverbindung SV, koaxial mit dem vorzugsweise stabförmigen Schwingteil 4 verbunden. Die Schraubverbindung SV kann im vorderen Endbereich des Hülsengehäuses 41 versenkt angeordnet und durch einen Gewindezapfen am hinteren Ende des Handstückschaftes 25 gebildet sein, der in ein koaxiales Gewindeloch im Schwingteil 4 eingeschraubt ist. Die Schraubverbindung SV kann beim Vorhandensein einer Lagerhülse 35 aus elastisch verformbaren Material gemäß Fig. 1 oder beim Vorhandensein eines Lagerringes 35b aus elastisch verformbaren Material vorgesehen sein, der das Schwingteil 4 in vergleichbarer Weise elastisch zentriert und in einer Ringnut einer im vorderen Endbereich der Griffhülse bzw. des Hülsengehäuses 41 eingesetzten Innenhülse sitzt. Außerhalb des Hülsengehäuses 41 oder der Lagerhülse 35 weist der Handstückschaft 25 ein Werkzeugangriffselement zum Fest- oder Losschrauben auf, z.B ein Sechskant. Bei der vorliegenden Ausgestaltung besteht der Handstückschaft 25 aus zwei koaxial hintereinander angeordneten Schaftteilen 25a, 25b, die z.B. muffenförmig ineinandergesteckt und aneinander befestigt sind, z.B. durch Kleben oder Preßsitz in der Muffenverbindung.

Um das Lösen des Werkzeugschaftes 8 zu erleichtern, kann auch die Haltevorrichtung 6 gem. Fig. 7 mit einer Vorrichtung 116 zum Ausdrücken des Werkzeugschafts 8 ausgebildet sein. Hierzu kann ein Betätigungsglied 91 ebenfalls in Form einer Betätigungshülse dienen, das auf dem vorderen Endbereich des Handstückschafts 25 axial verschiebbar gelagert ist. Dabei kann diese Betätigungshülse 92b vorderseitig durch eine Stirnwand 92c geschlossen sein, wobei eine zwischen der Stirnwand 92c und einem Federteller 132 angeordnete Druckfeder 133 die Betätigungshülse 92b in ihre Bereitschaftsstellung vorspannt, in der sie sich im Gegensatz zu der Ausgestaltung nach Fig. 4 in einer vorderseitigen Endstellung befindet, in der die vorderseitige Flanke der Kalotte 118 die Keilfläche 117 bildet. Bei einer Verschiebung der Betätigungshülse 92b gemäß Pfeil 115a nach hinten durch axialen Druck auf deren Stirnwand 92c werden die Sicherungsnase 125a aus der Sicherungsausnehmung 28 und der Werkzeugschaft 8 aus dem Steckloch 27 durch die Keilfläche 117 herausgedrückt. Um diese Bewegung zu ermöglichen, ist die Steckausnehmung 94 um das Maß der Bewegung nach vorne verlängert. In der Bereitschaftsstellung gemäß Fig. 7 ist die axiale Bewegung durch einen Anschlag 134 begrenzt, der durch ein oder zwei einander gegenüberliegend angeordnete Anschlagstifte 135 gebildet sein kann, die in radialen Löchern 136 des Handstückschaftes 25 radial verschiebbar gelagert sind, durch die Kraft einer Feder radial nach außen vorgespannt sind und jeweils in eine Längsnut 137 in der Innenmantelfläche der Betätigungshülse 92b einfassen, deren Länge L um das Maß der axialen Bewegung größer bemessen ist, als die zugehörige Querschnittsabmessung des Anschlagstiftes 134, so daß die Betätigungshülse 92b in ihre Längsstellung verschoben werden kann. Die Seitenflächen der Längsnuten 137 bilden mit den darin einfassenden Stiften 135 eine Drehsicherung für die Betätigungshülse 92b. Bei der vorliegenden Ausgestaltung ist jeweils der Anschlagstift 135 in einer topfförmigen Buchse 138 aus elastisch verformbaren Material wie Gummi oder Kunststoff aufgenommen, die in entsprechend größer bemessenen Löchern 139 sitzen.

Die Drehposition-Einstellvorrichtung 114 kann anstelle von Kalotten 31 im Schaft 8 durch eine Steckfassung 141 gebildet sein, wobei diese und der darin einsteckbare Werkzeugschaft 8 jeweils eine so unrunde und zueinander passende Querschnittsform aufweisen, daß der Werkzeugschaft 8 in zwei oder mehr zueinander verdrehten Stellungen darin einsteckbar ist. Beim Vorhandensein einer unrunden Steckfassung 141 kann anstelle von Kalotten 31 eine Ringnut entsprechender Queschnittsform angeordnet sein. Vorzugsweise weist die Querschnittsform der Steckfassung 141 und des Werkzeugschaftes 8 drei oder mehr, insbesondere sechs, regelmäßige Ecken auf. Die Querschnittsform kann auch im Sinne einer Vielzahnverbindung ausgebildet sein. Die Steckfassung 141 braucht sich nicht über die gesamte Länge des Stecklochs 27 erstrecken. Es reicht für eine gute Funktion aus, wenn nur ein Abschnitt oder der freie Endbereich des Werkzeugschafts 8 und ein passender innerer Abschnitt oder Endbereich 27b des Stecklochs 27 als Steckfassung 141 ausgebildet sind, wie es Fig. 8 und 9 zeigen. Bei dieser Ausgestaltung kann die Sicherungsausnehmung 28 in einfacher Weise als Ringnut ausgebildet sein.

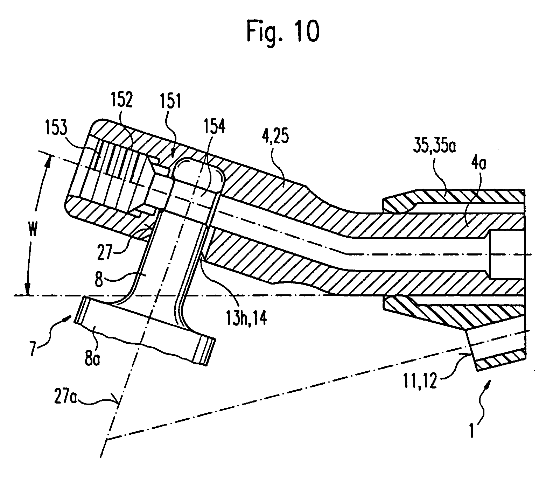

Bei der Ausgestaltung gem. Fig. 10, bei der gleiche oder vergleichbare Teile mit gleichen Bezugszeichen versehen sind, ist anstelle einer Verrastungsvorrichtung eine Schraubverbindung oder Klemmverbindung 151 mit einer vorzugsweise von vorne in ein Gewindeloch 152 des Handstückschafts 25 eingeschraubten Sicherungsschraube 153 vorgesehen, die in eine Ringnut 154 oder in eine von mehreren auf dem Umfang verteilt angeordneten Sicherungsausnehmungen des Werkzeugschafts 8 so einschraubbar ist, daß sie gegen den in das Steckloch 27 aufgenommenen Werkzeugschaft 8 drückt oder mit Drehbewegungsspiel in die jeweilige Ausnehmung einfaßt, wie es vorbeschrieben worden ist, um eine drehbare Halterung oder eine Drehposition-Einstellvorrichtung zu bilden. Auch bei dieser Ausgestaltung kann Behandlungsflüssigkeit durch einen radialen und/oder axialen Kanal im Werkzeugschaft 8 zu wenigstens einer der abrasiven Arbeitsflächen des Werkzeugs 7 geführt werden oder durch eine am Werkzeugschaft 8 oder in der Wandung des Stecklochs 27 angeordnete Längsnut 13h, die einen Austritt des Behandlungsmittels außenseitig am Werkzeug 7 ermöglicht.

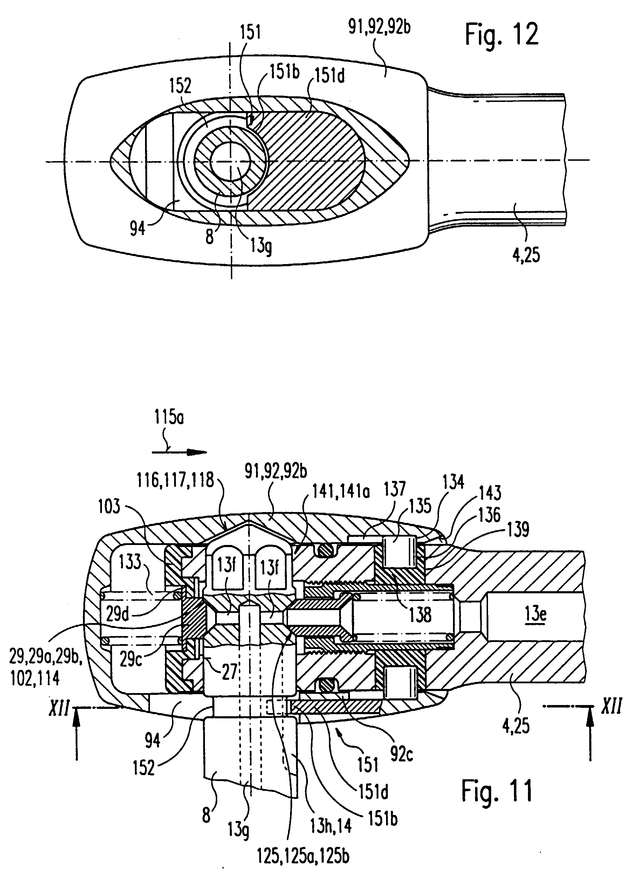

Die Fig. 11 und 12 zeigen mehrere Ausführungsbeispiele, die in Kombination oder jeweils einzeln angeordnet sein können, wobei gleiche oder vergleichbare Teile mit gleichen Bezugzeichen versehen sind.

Es ist eine Betätigungshülse 92a entsprechend dem Ausführungsbeispiel nach Fig. 7 vorgesehen, die in Richtung des Pfeiles 115a nach hinten zu verschieben ist, um den Werkzeugschaft 8 zu lösen, wie es vorbeschrieben worden ist. Es ist auch eine vorbeschriebene Steckfassung 141 zwischen dem Werkzeugschaft 8 und dem Handstückschaft 25 vorgesehen.

Zur axialen Halterung des Werkzeugschaftes 8 kann anstelle oder zusätzlich zur Verrastungsvorrichtung 125 eine Sicherungsnase 29b vorgesehen sein, die durch die Kraft einer Feder in eine Kalotte 31 oder Ringnut des Werkzeugschafts 8 einfaßt oder vorgespannt ist, jedoch unabhängig von einer Bewegung des Betätigungsgliedes 91 aus der Kalotte 31 oder der Ringnut entfernbar oder verdrängbar ist. Ausgehend von der Ausgestaltung gemäß Fig. 7 ist hierzu die Sicherungsnase 29b durch die Feder 133 in ihre Sicherungsstellung beaufschlagt, wobei die Sicherungsnase 29b einteilig am Federteller 103 angeformt sein kann oder als separates Bauteil an diesen anmontiert sein kann und vorzugsweise unmittelbar durch die Druckfeder 133 beaufschlagt sein kann. Bei der vorliegenden Ausgestaltung ist die Sicherungsnase 29b an einen prismatischen oder zylindrischen Zapfen 29c angeformt, der von vorne in ein Loch des Federtellers 132 eingesetzt ist und an seinem der Nase abgewandten Ende einen Flansch 29d aufweist, der an der dem Steckloch 27 abgewandten Seite des Federtellers 132 anliegt, vorzugsweise an der Stufenfläche eines Stufenlochs. Sowohl bei der Ausgestaltung gemäß Fig. 4 als auch bei der Ausgestaltung gemäß Fig. 11 kann die Sicherungsnase 29b anstelle einer konvex gerundeten Form Schrägflächen oder eine Kegelstumpffläche an einem nur geringfügig in der Kalotte 31 oder die Ringnut einfassenden Nasenstumpf aufweisen.

Es ist im weiteren vorteilhaft, am hinteren Rand der Betätigungshülse 92b in Flucht mit der oder den Längsnuten 137 jeweils eine nach hinten divergierende Einführungsrundung oder -schräge 143 einzuordnen, wodurch die Montage und die Demontage der Betätigungshülse 92b wesentlich erleichtert wird. Beim Aufschieben der Betätigungshülse 92b von vorne sorgt die Einführungsschräge 143 für ein selbsttätiges Einfedern des zugehörigen Anschlagstiftes 135, der hinter dem Anschlag 134 selbsttätig in die zugehörige Längsnut 137 einrastet. Wie Fig. 11 deutlich erkennen läßt, endet die Einführungsschräge 143 über der zugehörigen Buchse 138. Hierdurch ist es möglich, zum Demontieren der Betätigungshülse 92b mit einem spitzen Werkzeug, z.B. einem Schraubenzieher, in den Keil der Einführungsschräge 143 einzufassen und die Buchse 138 mit dem zugehörigen Anschlagstift 135 soweit einzuschieben, daß die Betätigungshülse 92b abgezogen werden kann.

Bei der Ausgestaltung nach Fig. 11 und 12 ist auch eine Verrastungsvorrichtung 151 zur Sicherung des Werkzeugschaftes 8 in seiner in die Haltevorrichtung 6 aufgenommenen Position, wobei diese Verrastungsvorrichtung 151 eine Sicherungsnase 151b an einem Verrastungs- bzw. Sicherungsteil 151d aufweist, daß fest mit dem Betätigungsglied 92, hier der Betätigungshülse 92b verbunden ist und somit mit dem Betätigungsschieber 92 bewegbar ist und eine Bewegungseinheit bildet. Diese Sicherungsnase 151b kann durch einen Quersteg am Betätigungsschieber 92 gebildet sein, der in der Bereitschaftsstellung in das Steckloch 27 bzw. in dessen Projektion hineinragt oder sich dazu sekantial erstreckt und in eine Verrastungsausnehmung 152 im Werkzeugschaft 8 einfaßt, bei der es sich um eine Ringnut handeln kann. Die Sicherungsnase 151b kann - längs des Stecklochs 27 gesehen - in Anpassung an die Querschnittsform des Ringnutgrundes konkav geformt, z.B. gerundet sein, so daß sie den Werkzeugschaft 8 sichelförmig zu umgreifen vermag. Der Sicherungsnase 151b gegenüberliegend ist die Steckausnehmung 94 für den Werkzeugschaft 8 verlängert, so daß der Betätigungsschieber 92 in seine nicht dargestellte, hier nach rechts verschobene, Lösestellung verschiebbar ist, in der die Sicherungsnase 151b aus der Sicherungsausnehmung 152 herausbewegt ist.

Diese, vorzugsweise an der Werkzeugseite des Handstückschafteschaftes angeordnete Verrastungsvorrichtung 151 kann anstelle oder zusätzlich zu der Verrastungsvorrichtung 102 und/oder 125 vorgesehen sein. Es kann auch die Vorrichtung 116 zum Herausdrücken des Werkzeugschaftes 8 vorgesehen sein. In diesem Falle werden beim Verschieben des Betätigungsschiebers 92 in seine Lösestellung die Sicherungsnase 151b direkt aus der Sicherungsausnehmung 152 und der Werkzeugschaft 8 durch die Keilfläche 117 gleichzeitig ausgeschoben.

Bei der vorliegenden Ausgestaltung ist die Sicherungsnase 151b durch eine flache Scheibe gebildet, die in einer der Steckausnehmung 94 seitlich benachbarten Ausnehmung 154 eingesetzt und befestigt ist, z.B. durch Kleben oder Schweißen. Die Ausnehmungs 154 kann weniger tief ausgebildet sein, als die Dicke der Umfangswand der Betätigungshülse 92a, so daß ein Wandteil 92c verbleibt, an der die Scheibe anliegen kann.

Der Handstückschaft 25 und die Teile der Haltevorrichtung 6 können aus korrosionsfestem Metall und/oder Kunststoff bestehen. Bei den vorbeschriebenen Ausführungsbeispielen bestehen die mit paralleler Schraffur dargestellten Teile aus Metall und die mit Kreuzschaffur versehenen Teile aus Kunststoff.

Die erfindungsgemäßen Ausgestaltungen ergeben auch eine vorteilhafte Werkzeugausgestaltung im Bereich eines Werkzeugschaftes 8. Dabei ist hervorzuheben, daß der Werkzeugschaft 8 Sicherungsausnehmungen aufweist, die einer axialen Sicherung und einer Drehsicherung dienen. Zur Drehsicherung dienen die Kalotten 31 und/oder der vorzugsweise am freien Ende des Werkzeugschafts 8 angeordnete Steckzapfen 141a am Werkzeugschaft 8 unrunden, z.B. sechskantigen Querschnitts. Bei einer regelmäßigen Anordnung dieses Mehrkants oder der Kalotten 31 läßt sich auch eine Drehposition-Einstellvorrichtung für das Werkzeug 7 verwirklichen, die die Anordnung des Werkzeugs 7 in unterschiedlichen Drehstellungen ermöglicht. Wenn ein mehrkantiger Steckzapfen 141a vorhanden ist, können anstelle von mehreren Kalotten 131 eine Ringnut im Werkzeugschaft 8 vorgesehen sein. Die Verrastungsausnehmung 152 kann bei einem Werkzeug 5, das nicht in unterschiedliche Drehpositionen eingestellt zu werden braucht, durch eine seitliche Ausnehmung gebildet sein. Für unterschiedliche Drehstellungen eignet sich eine Ringausnehmung besonders gut. Wenn ein Steckzapfen 141a mit unrundem Querschnitt als Drehsicherung vorhanden ist, kann die Sicherungsausnehmung 152 zusätzlich oder anstelle der Kalotten 31 oder einer sie bildenden Ringnut vorgesehen sein. Für die Zuführung des Behandlungs- oder Kühlmittels können der innere Kanalabschnitt 13g der innerhalb oder oberhalb einer oder mehrerer Arbeitsflächen des Werkzeugs 5 ausmündet, und/oder die äußere Längsnut 13h vorgesehen sein, die mit der sich axial im Handstückschaft erstreckenden Zuführungsleitung in Verbindung stehen. Eine oder zwei durch Abzweigungen gebildete Ausmündungsöffnungen 13i sind in Fig. 1 beispielhaft dargestellt.

Dem Behandlungsinstrument 1 können zwecks Durchführung unterschiedlicher Bearbeitungen unterschiedliche Werkzeuge 7 zugeordnet sein, mit denen es wahlweise bestückbar ist. Hierbei kann es sich um Werkzeuge 7 von z.B. unterschiedlicher Form und/oder Masse und/oder Beschaffenheit der abrasiven Arbeitsfläche, z. B. grob, mittel, fein, und/oder Werkzeuge 7 handeln, die für eine Zuführung eines Behandlungsmediums, z.B. Kühlflüssigkeit, eingerichtet sind oder nicht. Für solche unterschiedlichen Werkzeuge können unterschiedliche Leistungsanforderungen bestehen, um eine optimale Funktion oder Arbeitsleistung zu erreichen. Bei Werkzeugen 7 unterschiedlicher Masse kann dies dadurch bedingt sein, daß die Massenschwingung bei einer Leistungseinstellung spezifischer Größe besonders günstig ist, z.B. unter Berücksichtigung von Schwingungsresonanzen. Bei Werkzeugen 7 unterschiedlicher Beschaffenheit ihrer Arbeitsflächen, wie z.B. grob, mittel oder fein, ist ebenfalls eine Leistungseinstellung spezifischer Größe zwecks Verbesserung der Leistungsfähigkeit vorteilhaft. Außerdem ist bei diese Kriterium zu berücksichtigen, daß die Arbeitsflächen dazu neigen können, sich mit Spänen zuzusetzen und auch deshalb eine spezifische Leistungseinstellung vorteilhaft ist. Eine Leistungseinstellung in spezifischer Größe und ist auch bei Werkzeugen 7 vorteilhaft, die mit einer Kühlflüssigkeit oder trocken arbeiten. Es ist deshalb vorteilhaft, und dient einer einfacheren Handhabung, wenn am Behandlungsinstrument 1 Markierungen 155 für das Einstellglied 74 so angeordnet sind, daß bei Positionsübereinstimmung des Einstellgliedes 74 im Sinne der oder einer bestimmten Markierung eine Leistung eingestellt ist, die für ein zugeordnetes Werkzeug 7 mit einer korrespondierenden Markierung 156 vorteilhaft ist. Es kann sich um farbliche oder andere Markierungen, z.B. Symbole, handeln, denen jeweils ein zugehöriger Index 155a zur Postionsübereinstimmung auf dem Einstellweg am Behandlungsinstrument zugeordnet ist. Bei der vorliegenden Ausgestaltung können eine oder mehrere Markierungen 155 und ein oder mehrere zugehörige Indexe 155a am Einstellglied 74 und an der Griffhülse bzw. dem Hülsengehäuse 41 angeordnet sein, wie es vereinfacht dargestellt ist, z.B. in Form einer Skala, wobei an dem einen Teil die Markierungen 155 und an dem anderen Teil der wenigstens eine Index 155a angeordnet ist. An den Werkzeugen 7 kann jeweils die korrespondierende Markierung 156, z.B: am Werkzeugschaft 8 in der Nähe des Werkzeugkopfes, angeordnet sein. Unterschiedliche Farben eignen sich besonders gut, um ein Markierungs-Paar 155, 155a,156 von einem anderen Markierungs-Paar 155, 155a, 156 zu unterscheiden, wobei miteinander korrespondierende Markierunden 155, 155a, 156 vorzugsweise die gleiche Farbe aufweisen.

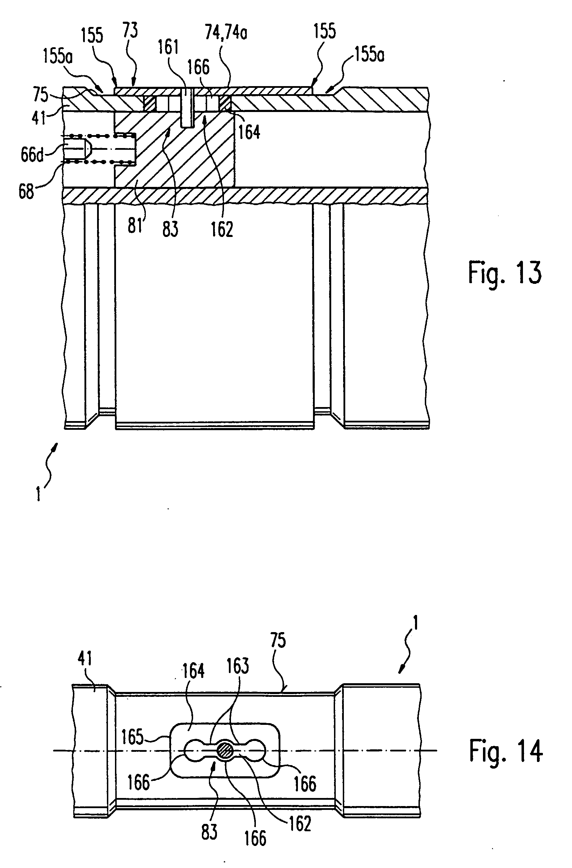

Beim Ausführungsbeispiel nach Fig. 13 und 14, bei dem gleiche oder vergleichbare Teile mit gleichen Bezugszeichen versehen, weist die Einstellvorrichtung 73 ein Einstellglied 74 vorzugsweise ebenfalls in Form einer Einstellhülse 74a auf, die in der entsprechend länger bemessenen Nut 75 axial verschiebar und in der jeweiligen Einstellposition durch die Feststellvorrichtung 83 stufenlos oder in Stufen feststellbar ist. Bei dieser Ausgestaltung ist das Verstellglied 81 durch einen radialen Verbindungsstift 161 starr mit dem Einstellglied 74 verbunden, wobei der Verbindungsstift 161 eine längs verlaufende Einstellnut 162 im Hülsengehäuse durchsetzt und wobei die längs gerichteten Nutwände 163 den Verbindungsstift 161 so stark zwischen sich klemmen, daß der Verbindungsstift nur mit einem manuellen Kraftaufwand axial verschiebbar ist, gegen eine unbeabsichtigte Verschiebung jedoch durch die Klemmwirkung der Nutwände 163 festgestellt ist. Hierzu kann die Einstellnut 162 in einem Einsatzteil 164 aus elastisch verformbaren Material, z.B. Kunststoff, ausgebildet sein, das in einer entsprechenden Ausnehmung 165 im Hülsengehäuse 41 sitzt. Falls bestimmte Einstellpunkte verwirklicht sein sollen, können in einer oder in beiden Nutwänden 163 Rastausnehmungen 166 angeordnet sein, in die der Verbindungsstift 161 einzurasten vermag. Bei der vorliegenden Ausgestaltung sind drei Raststellen axial hintereinander angeordnet. Um dies zu verdeutlichen, ist in Fig. 14 der dargestellte Längsabschnitt des Behandlungsinstruments 1 ohne die Einstellhülse 74a dargestellt.

Die Markierungen 155, 155a und 156 können bei dieser Ausgestaltung an der Vorderkante oder Hinterkante des Einstellglieds 74 und der daneben befindlichen Mantelfläche des Hülsengehäuses 41 angeordnet sein.

## Patentansprüche

1. Medizinisches oder dentalmedizinisches Behandlungsinstrument zur insbesondere spanabhebenden Behandlung von Körpergewebe oder einem Ersatzstoff, mit
- einem länglichen Handstück (1),
- das in seinem vorderen Endbereich eine Haltevorrichtung (6) mit einem Steckloch (27) aufweist, in das das Werkzeug (7) mit seinem Werkzeugschaft (8) einsteckbar und mittels eines Sicherungsteiles (29) sicherbar ist,
- das quer zum Werkzeugschaft (8) zwischen einer Sicherungsstellung, in der es in eine Sicherungsausnehmung (28) im Werkzeugschaft (8) einfaßt, und einer den Werkzeugschaft (8) freigebenden Freigabestellung bewegbar gelagert und durch die Kraft einer Feder in seine Sicherungsstellung beaufschlagt ist,
- wobei ein von außen manuell zugängliches Betätigungsglied (91) vorgesehen ist, das an der Haltevorrichtung (6) bewegbar gelagert ist und mit dem das Sicherungsteil (29) in seine Freigabestellung verschiebbar ist,
- und wobei das Betätigungsglied (91) quer zum Werkzeugschaft (8) verschiebbar gelagert ist.

2. Medizinisches oder dentalmedizinisches Behandlungsinstrument nach Anspruch 1, **dadurch gekennzeichnet,**
daß das Sicherungsteil (29) bewegbar oder starr mit dem Betätigungsglied (91) verbunden ist.

3. Medizinisches oder dentalmedizinisches Behandlungsinstrument zur insbesondere spanabhebenden Behandlung von Körpergewebe oder einem Ersatzstoff, mit
- einem länglichen Handstück (1),
- das in seinem vorderen Endbereich eine Haltevorrichtung (6) mit einem Steckloch (27) aufweist, in das das Werkzeug (7) mit seinem Werkzeugschaft (8) einsteckbar und mittels eines Sicherungsteiles (29) sicherbar ist,
- das quer zum Werkzeugschaft (8) zwischen einer Sicherungsstellung, in der es in eine Sicherungsausnehmung (28) im Werkzeugschaft (8) einfaßt, und einer den Werkzeugschaft (8) freigebenden Freigabestellung bewegbar gelagert und durch die Kraft einer Feder (103) in seine Sicherungsstellung beaufschlagt ist,
- wobei das Sicherungsteil (125a) bei einer axialen Verschiebung des Werkzeugschafts (8) mit einer über eine bestimmte Größe hinausgehenden Kraft aus der Sicherungsausnehmung (28) selbsttätig verdrängt wird,
- wobei ein von außen manuell zugängliches Betätigungsglied (91) vorgesehen ist, mit dem der Werkzeugschaft (8) verschiebbar ist,
- und wobei das Betätigungsglied (91) ein an der Haltevorrichtung (6) zwischen einer Haltestellung und einer Ausschubstellung bewegbar gelagertes Bauteil ist.

4. Medizinisches oder dentalmedizinisches Behandlungsinstrument nach Anspruch 3,
**dadurch gekennzeichnet,**
daß das Betätigungsglied (91) quer zum Werkzeugschaft (8) bewegbar ist.

5. Medizinisches oder dentalmedizinisches Behandlungsinstrument nach einem der vorherigen Ansprüche,
**dadurch gekennzeichnet,**
daß zwischen dem Betätigungsglied (91) und dem Werkzeugschaft (8) ein Untersetzungsgetriebe angeordnet ist.

6. Medizinisches oder dentalmedizinisches Behandlungsinstrument nach Anspruch 5,
**dadurch gekennzeichnet,**
daß das Untersetzunggetriebe ein Keilgetriebe mit einer schiefen Ebene oder Keilfläche (117), insbesondere ein Schubkeilgetriebe ist.

7. Medizinisches oder dentalmedizinisches Behandlungsinstrument nach einem der vorherigen Ansprüche,
**dadurch gekennzeichnet,**
daß das Betätigungsglied (91) auf der dem Werkzeug (7) abgewandten Seite und/oder auf einer oder beiden Seiten der Haltevorrichtung (6) angeordnet ist und/oder die Haltevorrichtung (6) nach vorne überragt.

8. Medizinisches oder dentalmedizinisches Behandlungsinstrumnet nach einem der vorherigen Ansprüche,
**dadurch gekennzeichnet,**
daß die Haltevorrichtung (6) an einem von einem Handstück (1) vorragenden Handstückschaft (25) angeordnet ist.

9. Medizinisches oder dentalmedizinisches Behandlungsinstrument nach Anspruch 8,
**dadurch gekennzeichnet,**
daß der Handstückschaft (25) ein quer verlaufendes Steckloch (27) für den Werkzeugschaft (8) aufweist.

10. Medizinisches oder dentalmedizinisches Behandlungsinstrument nach einem der vorherigen Ansprüche,
**dadurch gekennzeichnet,**
daß das Betätigungsglied (91) durch eine den Handstückschaft (25) umgebende Hülse (92a) gebildet ist.

11. Medizinisches oder dentalmedizinisches Behandlungsinstrument nach einem der vorherigen Ansprüche,
**dadurch gekennzeichnet,**
daß das Betätigungsglied (91) nach vorne oder nach hinten in seine Freigabestellung und/oder Ausstoßstellung bewegbar ist.

12. Medizinisches oder dentalmedizinisches Behandlungsinstrument nach Anspruch 10 oder 11,
**dadurch gekennzeichnet,**
daß die Hülse (92a) an ihrem vorderen Stirnende geschlossen ist.

13. Medizinisches oder dentalmedizinisches Behandlungsinstrument nach einem der vorherigen Ansprüche 10 bis 12,
**dadurch gekennzeichnet,**
daß das Sicherungsteil (29) an einem Querstift (96) der Hülse (92a) angeordnet ist, der den Handstückschaft (25) in einem Schlitz (28) durchsetzt.

14. Medizinisches oder dentalmedizinisches Behandlungsinstrument nach einem der vorherigen Ansprüche 9 bis 13,
**dadurch gekennzeichnet,**
daß sich das Steckloch (27) quer zum Handstückschaft (25) erstreckt.

15. Medizinisches oder dentalmedizinisches Behandlungswerkzeugnach einem der vorherigen Ansprüche 9 bis 14,
**dadurch gekennzeichnet,**
daß das Sicherungsteil (29) bezüglich des Stecklochs (27) vorderseitig und/oder rückseitig angeordnet ist.

16. Medizinisches oder dentalmedizinisches Behandlungswerkzeug nach einem der vorherigen Ansprüche 9 bis 15,
**daddurch gekennzeichnet,**
daß bezüglich des Stecklochs (27) vorzugsweise rückseitig ein Sicherungsteil (125) mit einer konvergenten Sicherungsnase (125a) vorgesehen ist, das quer zum Werkzeugschaft (8) zwischen einer Sicherungsstellung, in der es in eine Sicherungsausnehmung (28) im Werkzeugschaft (8) einfaßt, und einer den Werkzeugschaft (8) freigebenden Freigabestellung bewegbar gelagert und durch die Kraft einer Feder (126) in seine Sicherungsstellung beaufschlagt ist, wobei das Sicherungsteil (29) bei einer axialen Verschiebung des Werkzeugschafts (8) mit einer über eine bestimmte Größe hinausgehenden Kraft aus der Sicherungsausnehmung (28) selbsttätig verdrängt wird und vorzugsweise einen axialen Durchgangskanal (13i) aufweist.

17. Medizinisches oder dentalmedizinisches Behandlungsinstrument nach einem der vorherigen Ansprüche 6 bis 16,
**dadurch gekennzeichnet,**
daß die Keilfläche (117) durch eine Ausnehmung (118) im Betätigungsglied (91) gebildet ist.

18. Medizinisches oder dentalmedizinisches Behandlungsinstrument nach einem der vorherigen Ansprüche,
**dadurch gekennzeichnet,**
daß im freien Endbereich des Werkzeugschaftes (8) eine Kuppel oder eine Kugel (119) in einer Ausnehmung (121) angeordnet ist, die den Werkzeugschaft (8) etwas überragt.

19. Medizinisches oder dentalmedizinisches Behandlungsinstrument nach einem der vorherigen Ansprüche,
**dadurch gekennzeichnet,**
daß es einen Schwingungserreger (5) aufweist, wobei vorzugsweise die Leistung des Schwingungserregers (5) automatisch und/oder manuell einstellbar ist.

20. Werkzeug (7) insbesondere für ein Behandlungsinstrument (1) nach einem der vorherigen Ansprüche, mit einem Arbeitsabschnitt (8a) und einem damit verbundenen Werkzeugschaft (8), wobei der Werkzeugschaft (8) an seinem freien Ende einen Steckzapfen (141a) unrunder Querschnittsform und in einem davon zum Arbeitsabschnitt (8a) in gerichteten Abstand ein oder zwei axial voneinander beabstandete Ausnehmungen (28,152)aufweist.

21. Werkzeug nach Anspruch 20,
**dadurch gekennzeichnet,**
daß der Steckzapfen (141a) eine regelmäßige mehrkantige Querschnittsform aufweist, insbesondere eine vier- sechs- oder achtkantige Querschnittsform.

22. Werkzeug nach Anspruch 20 oder 21,
**dadurch gekennzeichnet**,
daß eine oder beide Ausnehmungen (28, 152) durch auf den Umfang verteilt angeordnete Kalotten (31) oder/oder durch eine Ringnut gebildet ist bzw. sind.

23. Werkzeug nach einem der vorherigen Ansprüche 20 bis 22,
**dadurch gekennzeichnet,**
daß im Werkzeugschaft (8) ein Medienkanal (13f,13g) angeordnet ist, der im Bereich einer der Ausnehmungen (28, 152) eine Eintrittsöffnung aufweist und im Arbeitsabschnitt (8a) oder in dessen Nahe ausmündet.

24. Werkzeug nach einem der vorherigen Ansprüche 20 bis 23,
**dadurch gekennzeichnet,**
daß in der Mantelfläche des Werkzeugschaftes (8) eine axiale Nut (13h) angeordnet ist, die in einem Abstand vom freien Ende des Werkzeugschaftes (8) beginnt und sich in Richtung auf den Arbeitsabschnitt (8a), insbesondere bis in dessen Nähe erstreckt.
